# EUROPEAN PATENT SPECIFICATION

(11) **EP 1 281 771 B1**
(45) Date of publication and mention of the grant of the patent: **22.03.2006**
(21) Application number: 02254973.7
(22) Date of filing: 16.07.2002
(51) Int. Cl.: C12Q 1/44, C07K 14/00, C12N 5/00, C12N 15/00, G01N 33/50

(54) **Phosphodiesterase 10A cell-based assay and sequences**
Auf Zellen gegründetes Phosphodiesterase-10A-Assay und Sequenzen
Essai pour Phosphodiestérase 10A basé sur les cellules et les séquences

(30) Priority: 31.07.2001 US 308978 P
(43) Date of publication of application: 05.02.2003
(73) Proprietor: Pfizer Products Inc., Groton, CT 06340-5146 (US)
(72) Inventor: James, Larry Carlton, Groton, Connecticut 06340 (US); Lebel, Lorraine Ann, Groton, Connecticut 06340 (US); Menniti, Frank Samuel, Groton, Connecticut 06340 (US); Strick, Christine Ann, Groton, Connecticut 06340 (US)
(74) Representative: Hayles, James Richard

(56) References cited:
- EP-A- 0 967 284
- FUJISHIGE KOTOMI ET AL: "Striatum- and testis-specific phosphodiesterase PDE10A: Isolation and characterization of a rat PDE10A" EUROPEAN JOURNAL OF BIOCHEMISTRY, vol. 266, no. 3, December 1999 (1999-12), pages 1118-1127, XP002258581 ISSN: 0014-2956
- SODERLING SCOTT H ET AL: "Isolation and characterization of a dual-substrate phosphodiesterase gene family: PDE10A" PROCEEDINGS OF THE NATIONAL ACADEMY OF SCIENCES OF THE UNITED STATES, vol. 96, no. 12, 8 June 1999 (1999-06-08), pages 7071-7076, XP002258583 June 8, 1999 ISSN: 0027-8424
- FUJISHIGE KOTOMI ET AL: "Cloning and characterization of a novel human phosphodiesterase that hydrolyzes both cAMP and cGMP (PDE10A)" JOURNAL OF BIOLOGICAL CHEMISTRY, vol. 274, no. 26, 25 June 1999 (1999-06-25), pages 18438-18445, XP002258582 ISSN: 0021-9258
- DATABASE BIOSIS [Online] BIOSCIENCES INFORMATION SERVICE, PHILADELPHIA, PA, US; 2002 KLEIMAN R J ET AL: "PDE10 REGULATION OF INTRACELLULAR SIGNALING IN STRIATAL MEDIUM SPINY NEURONS IN CULTURE." Database accession no. PREV200300268363 XP002258584 & SOCIETY FOR NEUROSCIENCE ABSTRACT VIEWER AND ITINERARY PLANNER, vol. 2002, 2002, page Abstract No. 43.18 32nd Annual Meeting of the Society for Neuroscience;Orlando, Florida, USA; November 02-07, 2002
- DATABASE BIOSIS [Online] BIOSCIENCES INFORMATION SERVICE, PHILADELPHIA, PA, US; 2002 SCHMIDT C J ET AL: "EFFECT OF PDE10 INHIBITION ON STRIATAL CYCLIC NUCLEOTIDE CONCENTRATIONS AND FUNCTION." Database accession no. PREV200300268364 XP002258585 & SOCIETY FOR NEUROSCIENCE ABSTRACT VIEWER AND ITINERARY PLANNER, vol. 2002, 2002, page Abstract No. 43.19 32nd Annual Meeting of the Society for Neuroscience;Orlando, Florida, USA; November 02-07, 2002

### SEQUENCE LISTING

<110> Pfizer Products Inc.
   James, Larry C.
   Lebel, Lorraine A.
   Menniti, Frank S.
   Strick, Christine A.
<120> PDE10 cell-Based Assay and Sequences
<130> PC23111ANIS
<150> us 60/308,978
   <151> 2001-07-31
<160> 15
<170> Patentin version 3.1
<210> 1
   <211> 3219
   <212> DNA
   <213> Rattus norvegicus
<400> 1
   gcatgagcag ccatggtgtg gaaagcagtc aagagaagag ctgccaggca gatgggccct      60
   gagctggagg tgcagcgtga aggatgcaca gctggagctg caagctgcag cccagatgtc     120
   tggtttgacg gatgaaaagg tgaaggccta tctttccctc catccccagg tattagacga     180
   gtttgtttct gaaagtgtta gtgcggagac cgtggagaag tggctgaaga ggaaaaacaa     240
   caaagcagaa gatgaaccat ctcctaagga agtcagcagg taccaggaca cgaacatgca     300
   gggagtcgtg tacgagctga acagctacat agagcagcgc ctggacaccg gcggggacaa     360
   ccacctgctc ctgtacgagc taagcagtat catcaggata gccacaaaag ccgacggatt     420
   tgcactgtac ttccttggag agtgcaataa tagtctgtgt gtcttcacac cacccggaat     480
   gaaggaaggt caaccccgtc tcatccccgc agggcccatc acccagggca ccaccatctc     540
   tgcctatgtg gccaagtcta ggaagaccct gctggtagag gacatccttg gggatgagcg     600
   atttcccaga ggcactggtc tggagtcagg aacccgaatc cagtctgtcc tttgcttgcc    660
   tattgtcact gccattggag acttgattgg catccttgaa ctgtacaggc actggggcaa    720
   agaggccttc tgcctcagcc atcaggaggt tgcaaccgcc aatctcgctt gggcttccgt    780
   agcaatacac caggtgcagg tgtgcagagg tctcgccaag cagaccgaac tgaatgactt    840
   cctgctcgat gtatcaaaga catactttga taacatagtc gccatagact ctctacttga    900
   acacatcatg atatatgcaa aaaatctagt gaacgccgac cgctgcgcgc tcttccaggt    960
   ggaccacaag aacaaggagc tgtactcgga cctgtttgac attggggagg agaaggaggg   1020
   gaagcccgtc ttcaagaaga ccaaggagat cagattttcc attgagaaag ggattgctgg   1080
   tcaagtggca agaacgggag aagtcctgaa cattcctgat gcctacgcag acccgcgctt   1140
   taacagggag gtggacctgt acacaggcta taccacgcgg aacattctgt gtatgcccat   1200
   agtgagccgc ggcagcgtga tcggtgtggt gcaaatggtt aacaagatca gcggcagcgc   1260
   cttctccaag acggatgaga acaacttcaa gatgtttgct gtcttctgcg ctctggccct   1320
   gcactgcgct aacatgtacc acaggatccg ccactcagag tgcatctaca gggttaccat   1380
   ggagaagctg tcttaccaca gcatctgcac ctctgaggaa tggcaaggcc tcatgcactt   1440
   caacttgcca gcacgcatct gccgggacat cgagctattc cactttgaca ttggtccttt   1500
   cgagaacatg tggcctggga tctttgtcta catgatccat cggtcttgtg ggacatcctg   1560
   ttttgaactt gaaaaattgt gccgttttat catgtctgtg aagaagaact ataggcgggt   1620
   tccttaccac aactggaagc atgcagtcac ggtggcgcac tgcatgtacg ccatacttca   1680
   aaacaacaat ggcctcttca cagaccttga gcgcaaaggc ctgctaattg cctgtctgtg   1740
   ccatgacctg gaccacaggg gcttcagtaa cagctacctg cagaaattcg accaccccct  1800
   ggctgcgttg tactccacct ccaccatgga gcaacaccac ttctcccaga cggtgtccat   1860
   cctccagctg gaaggacaca acatcttctc caccctgagc tccagcgagt acgagcaggt   1920
   gctggagatc atccgcaaag ccatcatcgc cactgacctc gcactgtact ttgggaacag   1980
   gaagcagttg gaggagatgt accagacagg gtcgctgaac ctccacaacc agtcccatcg   2040
   agaccgcgtc atcggcttga tgatgactgc ctgcgatctt tgctctgtga cgaaactatg   2100
   gccagttaca aaattgacag caaatgatat atatgcagag ttctgggctg agggggatga   2160
   gatgaagaag ttggggatac agcccatccc tatgatggac agagacaagc gagatgaagt   2220
   ccctcaagga cagcttggat tctacaatgc tgtggccatc ccctgctata ccaccctgac   2280
   gcagatcctc ccacccacag agcctctgct gaaggcctgc agggataacc tcaatcagtg   2340
   ggagaaggta attcgagggg aagagacagc aatgtggatt tcaggcccag caactagcaa   2400
   aagcacatct gagaagccga ccaggaaggt cgatgactga tcctgaggtg atgtctgcct   2460
   agcaactgac tcaacctgct tctgtgactt cgttcttttt atttttattt ttttaacggg   2520
   gtgaaaacct ctctcagaag gtaccgtcgc atatccatgt gaagcagatg actccctgcg   2580
   cacacctcgg accgtgagca acccgggctc caccgtgttc agacatcggc tattccatgg   2640
   ctccgcctga cccccgaatg ccatttgcta ccaggccaga actgcgctgg ctggaggggg 2700
   cagagacgac aggaggggtt cttacctgca tccttccatg agggtgtggt tctgtgtttc 2760
   atctctaaca gagatgctac tgcttggtgg cgtttgttag aaatgggaca cacgcccctg 2820
   tcgtgaagtt tacatgtgac cttcttgtag gtaacttgag ttcgtagcct gggacccctg 2880
   taatgaaggt tacagtccac aggtgataga gaaattcaag ctgtaagtta caggtgcact 2940
   ataagtgtgt tcattcagtt tacctggggg catggaggtg agtcagctcc acgaggaagg 3000
   aagcatacct ctgccctcat caaggggaca cagggtacat cccaggcatc agagaactgc 3060
   agctcacctc aaaccatgtc aaagaattaa aacacacccc catcccctca ctgtagcctt 3120
   tggcaacttc gccaaaccct tcacacaaag aaaataaaag taaggcgtat aaatttcctc 3180
   cagcaagcaa atcttgtgag taaaaaaaaa aaaaaaaaa 3219
<210> 2
   <211> 773
   <212> PRT
   <213> Rattus norvegicus
<400> 2
<210> 3
   <211> 23
   <212> DNA
   <213> Rattus norvegicus
<400> 3
   cccagtcacg acgttgtaaa acg 23
<210> 4
   <211> 23
   <212> DNA
   <213> Rattus norvegicus
<400> 4
   agcggataac aatttcacac agg 23
<210> 5
   <211> 22
   <212> DNA
   <213> Rattus norvegicus
<400> 5
   gtacagctcc ttgttcttgt gg 22
<210> 6
   <211> 22
   <212> DNA
   <213> Rattus norvegicus
<400> 6
   ccagcaatcc ctttctcaat gg 22
<210> 7
   <211> 22
   <212> DNA
   <213> Rattus norvegicus
<400> 7
   gtaggcatca ggaatgttca gg 22
<210> 8
   <211> 22
   <212> DNA
   <213> Rattus norvegicus
<400> 8
   tatgaccatg attacgccaa gc 22
<210> 9
   <211> 21
   <212> DNA
   <213> Rattus norvegicus
<400> 9
   gttgtaaaac gacggccagt g 21
<210> 10
   <211> 21
   <212> DNA
   <213> Rattus norvegicus
<400> 10
   tagcaataca ccaggtgcag g 21
<210> 11
   <211> 22
   <212> DNA
   <213> Rattus norvegicus
<400> 11
   cagaccgaac tgaatgactt cc 22
<210> 12
   <211> 33
   <212> DNA
   <213> Rattus norvegicus
<400> 12
   tgtcgactat aaatatgtct ggtttgacgg atg 33
<210> 13
   <211> 20
   <212> DNA
   <213> Rattus norvegicus
<400> 13
   ggaatgaagg aaggtcaacc 20
<210> 14
   <211> 20
   <212> DNA
   <213> Rattus norvegicus
<400> 14
   gctgtcaatt ttgtaactgg 20
<210> 15
   <211> 26
   <212> DNA
   <213> Rattus norvegicus
<400> 15
   agtcgacgtc atcgaccttc ctggtc 26

## Description

### Field of the Invention

The present invention provides methods for identifying agents that modulate PDE10A activity and the polynucleotide and polypeptide sequences for rat PDE10A.

### Background

Cyclic nucleotide phosphodiesterases (PDEs) catalyze the hydrolysis of the second messengers cAMP (cyclic adenosine 3'5'-monophosphate) and cGMP (cyclic guanine 3'5'-monophosphate) and play a pivotal regulatory role in a wide variety of signal transduction pathways (Beavo, Physiol. Rev. 75: 725-48, 1995). For example, PDEs mediate processes involved in vision (McLaughlin et al., Nat. Genet 4: 130-34, 1993), olfaction (Yan et al., Proc. Natl. Acad. Sci. USA 92: 9677-81, 1995), platelet aggregation (Dickinson et al., Biochem. J. 323: 371-77, 1997), aldosterone synthesis (MacFarland et al., J. Biol. Chem. 266: 136-42, 1991), insulin secretion (Zhao et al., J. Clin. Invest. 102: 869-73, 1998), T cell activation (Li et al., Science 283: 848-51, 1999), and smooth muscle relaxation (Boolell et al., Int. J. impot. Res. 8: 47-52, 1996; Ballard et al., J. Urol. 159: 2164-71, 1998).

PDEs form a superfamily of enzymes that are subdivided into 11 major families (Beavo, Physiol. Rev. 75: 725-48, 1995; Beavo et al., Mol. Pharmacol. 46: 399-05, 1994; Soderling et al., Proc. Natl. Acad. Sci. USA 95: 8991-96, 1998; Fisher et al., Biochem. Biophys. Res. Commun. 246: 570-77, 1998; Hayashi et al., Biochem. Biophys. Res. Commun. 250: 751-56, 1998; Soderling et al., J. Biol. Chem. 273: 15553-58, 1998; Fisher et al., J. Biol. Chem. 273: 15559-64. 1998; Soderting et al., Proc. Natl. Acad. Sci. USA 96: 7071-76, 1999; and Fawcett et al., Proc. Natl. Acad. Sci. USA 97: 3702-07, 2000).

Each PDE family is distinguished functionally by unique enzymatic characteristics and pharmacological profiles. In addition, each family exhibits distinct tissue, cellular, and subcellular expression patterns (Beavo et al., Mol. Pharmacol. 46: 399-405, 1994; Soderling et al., Proc. Natl. Acad. Sci. USA 95: 8991-96, 1998; Fisher et al., Biochem. Biophys. Res. Commun. 246: 570-77, 1998; Hayashi et al., Biochem. Biophys. Res. Commun. 250: 751-56, 1998; Soderling et al., J. Biol. Chem. 273: 15553-58, 1998; Fisher et al., J. Biol. Chem. 273: 15559-64, 1998; Soderling et al., Proc. Natl. Acad. Sci. USA 96: 7071-76, 1999; Fawcett et al., Proc. Natl. Acad. Sci. USA 97: 3702-07, 2000; Boolell et al., lnt. J. lmpot. Res. 8: 47-52, 1996; Ballard et al., J. Urol. 159: 2164-71, 1998; Houslay, Semin. Cell Dev. Biol. 9: 161-67, 1998; and Torphy et al., Pulm. Pharmacol. Ther. 12: 131-35, 1999). Accordingly, by administering a compound that selectively regulates the activity of one family or subfamily of PDE enzymes, it is possible to regulate cAMP and/or cGMP signal transduction pathways in a cell- or tissue-specific manner.

PDE10 is identified as a unique PDE based on primary amino acid sequence and distinct enzymatic activity. Homology screening of EST databases revealed PDE10A as the first member of the PDE10 family of phosphodiesterases (Fujishige et al., J. Biol. Chem. 274: 18438-18445, 1999; Loughney et al., Gene 234:109-117, 1999). The human, rat, and murine homologues have been doned and N-terminal splice variants have been identified for both the rat and human genes (Kotera et al., Biochem. Biophys. Res. Comm. 261: 551-557, 1999; Fujishige et al., Eur. J. Biochem. 266: 1118-1127, 1999; Soderling et al., Proc. Nati. Acad. Sd. USA 96: 7071-7076, 1999); there is a high degree of homology across species. PDE10A hydrolyzes cAMP and cGMP to AMP and GMP, respectively. The affinity of PDE1 0A for cAMP (Kₘ = 0.05 µM) is higher than for cGMP (Kₘ = 3 µM). However, the approximately 5-fold greater Vₘₐₓ for cGMP over cAMP has led to the suggestion that PDE10A is a unique cAMP-inhibited cGMPase (Fujishige et al., J. Biol. Chem. 274:18438-18445, 1999).

PDE10A is uniquely localized in mammals relative to other PDE families. Messenger RNA for PDE10A is highly expressed only in testis and brain (Lanfear and Robas, EP 0967284; Fujishige et al., Eur. J. Biochem. 266: 1118-1127, 1999; Soderling et al., Proc. Natl. Acad. Sci. USA 96: 7071-7076. 1999; Loughney et al., Gene 234:109-117, 1999). Initial studies indicated that, within the brain, expression is highest in the striatum (caudate and putamen), nucleus accumbens, and olfactory tubercle (Lanfear and Robas, *supra*). Accordingly, PDE10A selective modulation could be used to modulate levels of cydic nudeotides in these brain areas.

### Summary of the Invention

The present invention provides methods for identifying agents that selectively modulate PDE10A activity and the polynucleotide and polypeptide sequences for rat PDE10A.

In one aspect, the invention features a method of screening for an agent that inhibits intracellular phosphodiesterase 10A activity comprising administering the agent to striatal medium spiny neurons and submaximally activating adenylate cyclase, administering the agent to striatal medium spiny neurons and submaximally activating guanylate cyclase, measuring cAMP generation and cGMP generation, respectively, and calculating the cAMP EC₂₀₀ and the cGMP EC₂₀₀, respectively, wherein the agent is identified as a PDE10A inhibitor if the ratio of cAMP EC₂₀₀/ cGMP EC₂₀₀ is comparable to the ratio produced by administration of papaverine under the same assay conditions.

Preferably, the striatal medium spiny neurons are prepared as primary cultured neurons, adenylate cyclase is activated by forskolin, guanylate cyclase is activated by sodium nitroprusside, and the cAMP EC₂₀₀/cGMP EC₂₀₀ ratio ranges from 1.75-5.25, more preferably, from 3.0-4.0. Preferably, the concentration of cAMP and cGMP is measured by scintillation proximity assay. It is preferred that the neurons used to assess cAMP and cGMP are in separate samples. In addition, it is preferred that the agent is first identified *in vitro* as a PDE10A selective inhibitor. Alternatively, the agent is further identified as a PDE10A selective inhibitor by *in vitro* assay.

In another aspect, the invention features an isolated or purified polypeptide comprising the amino acid sequence of SEQ ID NO: 2

In a related aspect, the invention features an isolated or purified potynudeotide comprising a nucleic acid sequence encoding the polypeptide of SEQ ID NO: 2 and/or the coding sequence of SEQ ID NO: 1.

The invention also features a vector comprising the coding sequence of SEQ ID NO: 1, and a host cell expressing the coding sequence of SEQ ID NO: 1.

In addition, the invention provides a method of identifying an agent that modulates PDE10A activity, comprising contacting the agent with a rat PDE10A polypeptide comprising SEQ ID NO: 2 and measuring the activity of the PDE10A polypeptide, wherein a difference between the PDE10A polypeptide activity in the presence of the agent and in the absence of the agent is indicative that the agent modulates PDE10A activity.

Also featured by the invention is a method of identifying an agent that modulates PDE10A activity, comprising contacting the agent with a host cell expressing the coding sequence of SEQ ID NO: 1 and measuring the activity of the PDE10A polypeptide expressed by SEQ ID NO: 1, wherein a difference between the PDE10A polypeptide activity in the presence of the agent and in the absence of the agent is indicative that the agent modulates PDE10A activity.

Those skilled in the art will fully understand the terms used herein in the description and the appendant claims to describe the present invention. Nonetheless, unless otherwise provided herein, the following terms are as described immediately below.

An "agent that increases PDE10A activity" refers to a molecule which intensifies or mimics the biological activity of a PDE10A polypeptide. Such agents (i.e., agonists) may include proteins, nucleic acids, carbohydrates, small molecules, or any other compound or composition which increases the activity of a PDE10A either by increasing the amount of PDE10A present in a cell or by increasing the catalytic activity of a PDE10A polypeptide.

An "agent that decreases PDE10A activity" refers to a molecule which inhibits or attenuates the biological activity of a PDE10A polypeptide. Such agents (i.e., antagonists) may include proteins such as anti-PDE10A antibodies, nucleic acids, carbohydrates, small molecules, or any other compound or composition which decreases the activity of a PDE10A polypeptide either by reducing the amount of PDE10A polypeptide present in a cell, or by decreasing the catalytic activity of a PDE10A polypeptide.

An "allelic variant" is an alternative form of the gene encoding a PDE10A polypeptide. Allelic variants may result from at least one mutation in the nucleic acid sequence and may result in altered mRNAs or in polypeptides whose structure or function may or may not be altered. A gene may have none, one, or many allelic variants of its naturally occurring form. Common mutational changes which give rise to allelic variants are generally ascribed to naturally-occurring deletions, additions, or substitutions of nucleotides. Each of these types of changes may occur alone, or in combination with the others, one or more times in a given sequence.

An "altered" nucleic acid sequence encoding a PDE10A polypeptide includes a sequence with a deletion, insertion, or substitution of different nucleotides, resulting in a polypeptide with at least one functional characteristic of a PDE10A polypeptide. Included within this definition are polymorphisms which may or may not be readily detectable using a particular oligonucleotide probe of the polynucleotide encoding a PDE10A polypeptide. The encoded protein may also be "altered," and may contain one or more deletions, insertions, or substitutions of amino acid residues which produce a silent change and result in a PDE10A polypeptide that is substantially equivalent functionally to a known PDE10A polypeptide. Deliberate amino acid substitutions may be made on the basis of similarity in polarity, charge, solubility, hydrophobicity, hydrophilicity, and/or the amphipathic nature of the residues, as long as PDE10A polypeptide is substantially functionally equivalent, e.g., in catalytic or immunologic activity

"Amplification" relates to the production of additional copies of a nucleic acid sequence. It is generally carried out using polymerase chain reaction (PCR) technologies well known in the art.

A cAMP EC₂₀₀/cGMP EC₂₀₀ value is "comparable" to that produced by papaverine if it varies by less than or equal to 50% of the papaverine value.

A "composition" comprising a given polynucleotide or polypeptide may comprise a dry formulation or an aqueous solution.

"Conservative amino acid substitutions" are those substitutions that, when made, least interfere with the properties of the original protein, i.e., the structure and especially the function of the protein is conserved and not significantly changed by such substitutions. Examples of conservative amino acid substitutions include the following: Ala replaced with Gly or Ser; Arg replaced with His or Lys; Asn replaced with Asp, Gin, or His; Asp replaced with Asn or Glu; Cys replaced with Ala or Ser, Gln replaced with Asn, Glu, or His; Glu replaced with Asp, Gin, or His; Gly replaced with Ala; His replaced with Asn, Arg, Gln, or Glu; Ile replaced with Leu or Val; Leu replaced with Ile or Val; Lys replaced with Arg, Gln, or Glu; Met replaced with Leu or Ile; Phe replaced with His, Met, Leu, Trp, or Tyr; Ser replaced with Cys or Thr; Thr replaced with Ser or Val; Trp replaced with Phe or Tyr, Tyr replaced with His, Phe, or Trp; and Val replaced with Ile, Leu, or Thr. Conservative amino acid substitutions generally maintain the same, or essentially the same (a) structure of the polypeptide backbone in the area of the substitution, for example, as a beta sheet or alpha helical conformation, (b) charge or hydrophobicity of the molecule at the site of the substitution, and/or (c) bulk of the side chain.

The term "derivative" refers to the chemical modification of a polypeptide or polynucleotide sequence. Chemical modifications of a polynucleotide sequence can include, for example, replacement of hydrogen by an alkyl, acyl, hydroxyl, or amino group. A derivative polynucleotide encodes a polypeptide which retains at least one biological or immunological function of the natural molecule. A derivative polypeptide is one modified by glycosylation, pegylation, or any other process that retains at least one biological or immunological function of the polypeptide from which it was derived.

A "fragment" is a unique portion of a PDE10A polypeptide or the polynucleotide encoding a PDE10A polypeptide which is identical in sequence to, but shorter in length than, the parent sequence. A fragment used as a probe, primer, antigen, therapeutic molecule, or for other purposes, may be at least 5, 10, 15, 20, 25, 30, 40, 50, 60, 75, 100, 150, 250, or at least 500 contiguous nucleotides or amino acid residues in length. Fragments may be preferentially selected from, or lack, certain regions of a molecule.

The term "identity" refers to a degree of complementarity. There may be partial similarity or complete identity. The word "similarity" may substitute for the word "identity." A partially complementary sequence that at least partially inhibits an identical sequence from hybridizing to a target nucleic acid is referred to as "substantially similar." The inhibition of hybridization of the completely complementary sequence to the target sequence may be examined using a hybridization assay (Southern or Northern blot, solution hybridization, and the like) under conditions of reduced stringency. A substantially similar sequence or hybridization probe will compete for and inhibit the binding of a completely similar (identical) sequence to the target sequence under conditions of reduced stringency. This is not to say that conditions of reduced stringency are such that non-specific binding is permitted. Rather, reduced stringency conditions require that the binding of two sequences to one another be a specific (i.e.. a selective) interaction. The absence of non-speafic binding may be tested by the use of a second target sequence which lacks even a partial degree of complementarity (e.g., less than about 30% similarity or identity). In the absence of non-specific binding, the substantially similar sequence or probe will not hybridize to the second non-complementary target sequence.

The phrases "percent identity" and "% identity," as applied to potynudeotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Percent identity between polynucleotide sequences may be determined using the default parameters of the CLUSTAL V algorithm as incorporated into the MegAlign® version 3.12e sequence alignment program. This program is part of the LASERGENE software package, a suite of molecular biological analysis programs (DNASTAR, Madison, WI). CLUSTAL V is described in Higgins and Sharp, CABIOS 5:151-153, 1989, and in Higgins et al., CABIOS 8:189-19, 1992. Percent identity is reported by CLUSTAL V as the "percent similarity" between aligned polynucleotide sequence pairs. Alternatively, a suite of commonly used and freely available sequence comparison algorithms is provided by the National Center for Biotechnology Information (NCBI) Basic Local Alignment Search Tool (BLAST) (Altschul et al., J. Mol. Biol. 215:403- 410, 1990), which is available from several sources, including the NCBI, Bethesda, MD, and at http://www.ncbi.nim.nih.gov/blast/. The BLAST software suite includes various sequence analysis programs including "blastn," that are used to align a known polynudeotide sequence with other potynudeotide sequences from a variety of databases. Also available is a tool called "BLAST 2 Sequences" that is used for direct pairwise comparison of two nudeotide sequences. "BLAST 2 Sequences" can be accessed and used interactively at http:/www.ncbi.nlm.nih.gov/blast/bl2seq/bl2.html. The "BLAST 2 Sequences" tool can be used for both blastn and blastp (discussed below). BLAST programs are commonly used with gap and other parameters set to default settings. For example, to compare two nucleotide sequences, one may use blastn with the "BLAST 2 Sequences" tool Version 2.0.9 (May-07-1999) using either Blossum 62 matrix or PAM250 matrix, a gap weight of 40, 50, 60, 70, or 80, and a length weight of 1, 2, 3, 4, 5, or 6. Percent identity may be measured over the length of an entire defined sequence, for example, as defined by a particular SEQ ID number, or may be measured over a shorter length, for example, over the length of a fragment taken from a larger, defined sequence, for instance, a fragment of at least 20, at least 30, at least 40, at least 50, at least 70, at least 100, or at least 200 contiguous nucleotides. Such lengths are exemplary only, and it is understood that any fragment length disdosed by the sequences shown herein may be used to describe a length over which percentage identity may be measured. Nudeic acid sequences that do not show a high degree of identity may nevertheless encode similar amino acid sequences due to the degeneracy of the genetic code. It is understood that changes in a nucleic acid sequence can be made using this degeneracy to produce multiple nucleic acid sequences encompassed by the invention that all encode the same or substantially the same PDE10A polypeptide.

The phrases "percent identity" and "% identity," as applied to polypeptide sequences, refer to the percentage of residue matches between at least two polypeptide sequences aligned using a standardized algorithm. Methods of polypeptide sequence alignment are well known. Some alignment methods take into account conservative amino acid substitutions. Such conservative substitutions, explained in more detail above, generally preserve the hydrophobicity and acidity at the site of substitution, thus preserving the structure and function of the polypeptide. Percent identity between polypeptide sequences may be determined using the default parameters of the CLUSTAL V algorithm as incorporated into the MegAlign® sequence alignment program (DNASTAR, Madison, WI). The PAM250 matrix is selected as the default residue weight table. As with polynucleotide alignments, the percent identity is reported by CLUSTAL V as the "percent similarity" between aligned polypeptide sequence pairs.

Alternatively, the NCBI BLAST software suite may be used. For example, for a pairwise comparison of two polypeptide sequences, one may use the "BLAST 2 Sequences" tool Version 2.0.9 (May-07-1999) with blastp set at default parameters. Such default parameters may be, for example, using Blossum 62 Matrix, score = 50, and word length = 3. Percent identity may be measured over the length of an entire defined polypeptide sequence, for example, as defined by a particular SEQ ID number, or may be measured over a shorter length, for example, over the length of a fragment taken from a larger, defined polypeptide sequence, for instance, a fragment of at least 15, at least 20, at least 30, at least 40, at least 50, at least 70, or at least 100 contiguous residues. Such lengths are exemplary only, and it is understood that any fragment length supported by the sequences shown herein, including the Figures and Sequence Listing, may be used to describe a length over which percentage identity may be measured.

By a "host" is meant a transgenic cell (e.g., mammalian, bacterial, insect) or an animal (e.g., non-human mammal) that is transfected with, and capable of expressing, a heterologous polynucleotide.

A "heterologous" polynucleotide is one which is foreign, or non-naturally occurring, or non-naturally positioned in the genome of the host cell.

"Hybridization" refers to the process by which a polynucleotide strand anneals with a complementary strand through base pairing under defined hybridization conditions. Specific hybridization is an indication that two nucleic acid sequences share a high degree of identity. Specific hybridization complexes form under permissive annealing conditions and remain hybridized after the "washing" step(s). The washing step(s) is (are) particularly important in determining the stringency of the hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments, whereas wash conditions may be varied among experiments to achieve the desired stringency, and therefore hybridization specificity. Permissive annealing conditions occur, for example, at 68°C in the presence of about 6X SSC, about 1% (w/v) SDS, and about 100 pg/ml denatured salmon sperm DNA.

Generally, stringency of hybridization is expressed, in part, with reference to the temperature under which the wash step is carried out. Generally, such wash temperatures are selected to be about 5°C to 20°C lower than the thermal melting point (Tm) for the specific sequence at a defined ionic strength and pH. The Tm is the temperature (under defined ionic strength and pH) at which 50% of the target sequence hybridizes to a perfectly matched probe. An equation for calculating Tm and conditions for nucleic acid hybridization are well known and can be found in Sambrook et al., 1989, *Molecular Cloning: A Laboratory Manual,* 2^{nd} ed., Vol. 1-3, Cold Spring Harbor Press, Plainview, NY; specifically see Vol. 2, chapter 9.

High stringency conditions for hybridization between polynucleotides of the present invention include wash conditions of about 55-68°C in the presence of about 0.2-1.0X SSC and about 0.1% SDS, for about 1 hour.

In general, hybridization reactions can be carried out at temperatures of about 65°C, 60°C, 55°C, or 42°C. SSC concentration may be varied from about 0.1 to 2X SSC, with SDS being present at about 0.1%. Typically, blocking reagents are used to block non-specific hybridization. Such blocking reagents include, for instance, denatured salmon sperm DNA at about 100-200 µg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art. Hybridization, particularly under high stringency conditions, is suggestive of evolutionary similarity between the nucleotides, which is strongly indicative of a similar role for the nucleotides and their encoded polypeptides.

The term "hybridization complex" refers to a complex formed between two nucleic acid sequences by virtue of the formation of hydrogen bonds between complementary bases. A hybridization complex may be formed between sequences present in solution or formed between one nucleic acid sequence present in solution and another nucleic acid sequence immobilized on a solid support (e.g., paper, membranes, filters, chips, pins or glass slides).

By "isolated or purified'' is meant changed from the natural state "by the hand of man." If a polynucleotide or polypeptide exists in nature, then it is "isolated or purified" if it is changed and/or removed from its original environment For example, an "isolated or purified" polynucleotide is separated from other polynucleotides with which it is associated in nature. For example, a cDNA sequence that is removed from intronic sequence normally associated with the coding sequence is "isolated or purified." An "isolated or purified" polynucleotide sequence may be introduced into host cells in culture or in whole organisms for transient or stable expression and still be "isolated and purified," because the polynucleotide would not be in its naturally occurring form or environment. However, polynucleotide sequences as members of cDNA libraries are excluded from what is meant by "isolated or purified." An "isolated or purified" polypeptide is separated from at least one cellular component with which it is associated in nature. Preferably, the polypeptide is at least 60% free, more preferably, at least 75% free, and, most preferably, at least 90% free from other components.

By "modulates" is meant increases or decreases (including a complete elimination).

"Operably linked" refers to the situation in which a first nucleic acid sequence is placed in a functional relationship with a second nucleic acid sequence. For example, a promoter is operably linked to a coding sequence if the promoter functions to regulate transcription of the coding sequence. Generally, operably linked DNA sequences may be in close proximity or contiguous and, where necessary to join two protein coding regions, in the same reading frame.

"Polynucleotide" generally refers to any RNA (e.g., mRNA). RNA-like, DNA (e.g., cDNA or genomic), or DNA like sequences, including, without limit, single-stranded, double-stranded, and triple-stranded sequences, sense or antisense strands, sequences generated using nucleotide analogs, hybrid molecules comprising RNA and DNA, and RNA or DNA containing modified bases. The polynucleotide can be naturally-occurring or synthesized.

The term "polypeptide" refers to an amino acid sequence, oligopeptide, peptide, polypeptide, or protein sequence, or a fragment of any of these, and to naturally occurring or synthetic molecules. It includes amino acid sequences modified either by natural processes, such as post-translational processing, or by chemical modifications well known in the art (see, e.g., *Proteins - Structure and Molecular Properties,* Ed. Creighton, W.H. Freeman and Co., New York, NY, 2^{nd} Ed, 1993; *Posttranslational Covalent Modification of Proteins,* Ed. Johnson, Academic Press, New York, NY, 1983; Seifter et al., Meth. Enzymol., 182: 626-46, 1990; and Rattan et al., Ann. NY Acad. Sci. 663: 48-62,1992). Known modifications include, but are not limited to, acetylation, acylation, ADP-ribosylation, amidation, covalent attachment of flavin, heme moiety covalent attachment, covalent attachment of a nucleotide or nudeotide derivative, lipid or lipid derivative, or phosphotidylinositol, cross linking, cydization, disulfide bond formation, demethylation, formation of cystine or pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer RNA-mediated addition of amino acids to proteins, such as arginylation and ubiquifination.

By "PDE10A activity" is meant PDE10A-mediated hydrolysis of cAMP and/or cGMP *in vitro, in vivo,* or *in situ.*

By a "selective" PDE10A inhibitor is meant an agent that inhibits PDE10A activity with an IC50 at least 10-fold less than that observed for inhibition of other PDEs.

A "substitution" refers to the replacement of one or more amino acids or nucleotides by different amino acids or nucleotides, respectively.

By "submaximally" activating adenylate cyclase or guanylate cyclase is meant administering a compound at a concentration that achieves an increase in cAMP or cGMP, respectively, that is about 10-50%, preferably, about 20-25%, of the value produced by maximally effective concentration of the compound.

"Transformation" or "transfection" describes a process of genetic modification by which heterologous DNA enters and renders a recipient cell capable of expressing the heterologous DNA. Transformation may occur in a prokaryotic or eukaryotic host cell according to various methods well known in the art The method is selected based on the type of host cell being transformed and includes, but is not limited to, viral infection, electroporation, heat shock, lipofection, and partide bombardment The terms "transformed cells" or "transfected cells" indude stably transformed cells in which the inserted DNA is capable of replication either as an autonomously replicating plasmid or as part of the host chromosome, as well as transiently transformed or transfected cells which express the inserted DNA or RNA for limited periods of time. All of such transformed or transfected cells are referred to as "transgenic."

A "variant" of a particular nucleic acid sequence is defined as a nucleic acid sequence having at least 40% sequence identity to the particular nucleic acid sequence over a certain length of one of the nucleic acid sequences using blastn with the "BLAST 2 Sequences" tool Version 2.0.9, set at default parameters. Such sequences may show, for example, at least 50%, at least 60%, at least 70%, at least 80%, at least 85%, at least 90%, at least 95%, or at least 98%, or greater, sequence identity over a certain defined length. A variant may be described as, for example, an "allelic" (as defined above), "splice," "species," or "polymorphic" variant. A splice variant may have significant identity to a reference molecule, but will generally have a greater or lesser number of polynucleotides due to alternate splicing of exons during mRNA processing. The corresponding polypeptide may possess additional functional domains or lack domains that are present in the reference molecule. Species variants are polynucleotide sequences that vary from one species to another. The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one nucleotide base. The presence of SNPs may be indicative of, for example, a certain population, a disease state, or a propensity for a disease state.

Other features and advantages of the invention will be apparent from the following detailed description and from the claims. While the invention is described in connection with specific embodiments, it will be understood that other changes and modifications that may be practiced are also part of this invention and are also within the scope of the appendant claims. This specification is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al., Basic *Methods in Molecular Biology,* Elsevir Sciences Publishing, Inc., New York, NY, 1986; Hames et at., *Nucleic Acid Hybridization,* IL Press, 1985; *Molecular Cloning,* Sambrook et al., *Current Protocols in Molecular Biology.* Eds. Ausubel et at., John Wiley and Sons; *Current Protocols in Human Genetics,* Eds. Dracopoli et al., John Wiley and Sons; *Current Protocols in Protein Science,* Eds. John E. Coligan et al., John Wiley and Sons; and *Current Protocols in Immunology,* Eds. John E. Coligan et at., John Wiley and Sons). All publications mentioned herein are incorporated by reference in their entireties.

### Description of the Figure

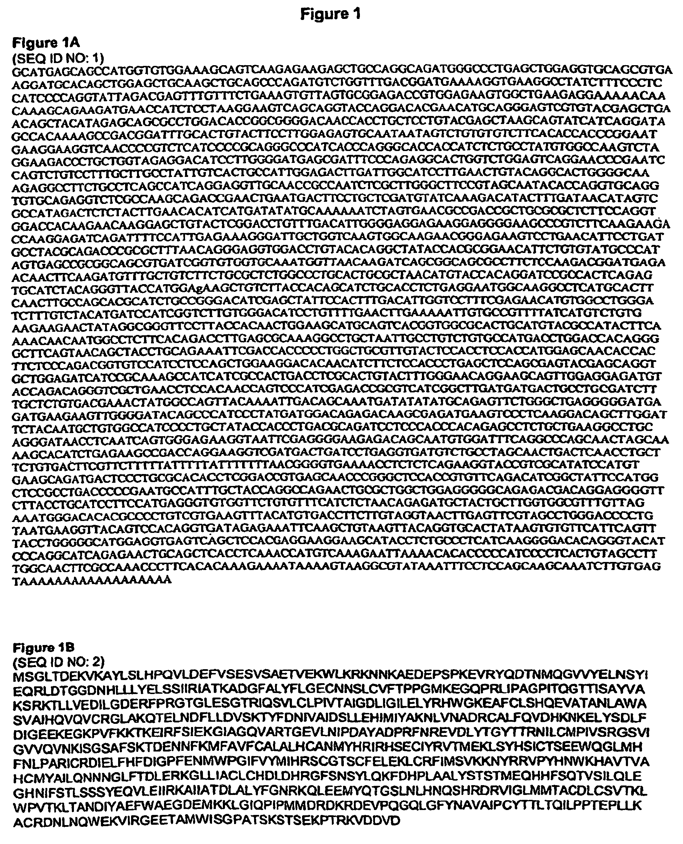

Figure 1A shows the polynucleotide sequence encoding rat PDE10A (Sea ID NO: 1). Figure 1B shows the amino acid sequence for the rat PDE10A polypeptide (SEQ ID NO: 2).

### Detailed Description

The present invention provides a cell-based screening assay using sfiatal medium spiny neurons to identify agents that inhibit PDE10A activity at the cellular level. Given the confirmed high level of PDE10A in striatal medium spiny neurons (as further described in Example 1 below), these cells are excellent candidates for use in a cell-based assay for identifying inhibitors of PDE10A activity. Such inhibitors are useful, for example, to treat disorders of movement or mood, anxiety, psychosis, drug addiction, and disorders of symptom deficient cognition, as further described in U.S. provisional application 60/285.148. The present invention also features rat PDE10A polynucleotide and polypeptide sequences.

### Cell-based Assay to Identify PDE10A Inhibitors

The cell-based assay of the invention stems from two discoveries further described in the Examples below. First, papaverine is a PDE10A selective inhibitor. And second, the administration of papaverine to striatal medium spiny neurons produces a unique profile of changes to levels of cAMP and cGMP. This unique profile indicates that other agents that produce the same combination of changes to cyclic nudeotides in striatal medium spiny neurons are also identified as PDE10A inhibitors.

To conduct the assay, mammalian striatal medium spiny neurons are used. The cells can be prepared as a primary culture (see, e.g., Ventimiglia et al., Eur. J. Neurosci. 7: 213-22, 1995). Alternatively, a striatal medium spiny neuron immortalized cell line can be used (Ehlich et al., Exp. Neurol. 167: 215-26, 2001; Cattaneo and Conti, J. Neuroscience Res. 53: 223-34, 1998; Wainvvright et al., J. Neuroscience 15: 676-88, 1995).

For primary cell culture, the neurons are prepared by dissecting brains from a mammalian embryo (e.g., an E17 rat or mouse embryo), dissociating the tissue into single cell suspension, and plating cells into appropriate vessels, such as multi-well plates. If desired, the presence of striatal medium spiny neurons in the preparation can be determined by testing GABA immunoreactivity (Ventimiglia et al., *supra*) and the expression of PDE10A can be confirmed, for example, by Western blot or RNase protection assay (see Example 3 below).

One example of the assay protocol is as follows. A primary cell culture of striatal medium spiny neurons (e.g., rat) at 4-5 day *in vitro* is washed in Ca²⁺/Mg²⁺ free phosphate buffered saline and preincubated for approximately 1 hour in phosphate buffered saline containing 30 mM HEPES, pH 7.4, 1 mM CaCl₂, 1 mg/ml dextrose, and 5 mM MgCl₂. Test agents are then incubated with the cells in the same buffer at 37°C for approximately 20 min.

To test for changes in cAMP, the neuron culture is also incubated with a submaximal concentration of a compound that stimulates adenylate cyclase (e.g., 1 µM forskolin). To test for changes in cGMP. the neuron culture is incubated with a submaximal concentration of a compound that stimulates guanylate cydase (e.g., 100 µM sodium nitroprusside (SNP)). A submaximal concentration for a compound that stimulates the generation of cyclic nucleotides is a concentration that generates 10-50%, preferably 20-25%, of the maximally effective concentration. The compound can be administered during the test agent incubation or for a period subsequent to the test agent incubation (e.g., 1-5 min.)

Cells are lysed and the appropriate cAMP and cGMP levels are measured in the cell lysate using standard methods. For example, the cAMP and cGMP levels can be measured using scintillation proximity assay (SPA) kits (Cat. No. RPA 540 and RPA 559, respectively, Amersham, Piscataway, NJ). Test agents are studied at varying concentrations such that the EC₂₀₀ value can be determined. The EC₂₀₀ is defined as the concentration of test agent which increases cAMP or cGMP levels by 200% as compared to the level of cAMP or cGMP measured in lysates from cells not treated by the test agent. Ideally, separate samples of cells are used to stimulate the cyclases and assess cyclic nucleotide levels. However, stimulation of adenylate cyclase and guanylate cyclase can be conducted in a single sample of cells, the cell lysate can be divided into two samples, and then cAMP and cGMP can be measured separately in these two lysates.

When using a primary culture of striatal medium spiny neurons and the exemplary assay conditions described above, a test agent is identified as a PDE10A inhibitor if it causes an increase in cAMP and cGMP, wherein the ratio of cAMP EC₂₀₀/cGMP EC₂₀₀ ranges from 1.75-5.25, preferably, 2.5-4.5, more preferably, 3.0-4.0. If the particular cells or assay conditions are varied from above, then the range of cAMP EC₂₀₀/cGMP EC₂₀₀ that indicates the agent is a PDE10A inhibitor can be determined by testing papaverine as a positive control under the appropriate conditions. An agent is identifed as a PDE10A inhibitor if it produces a ratio of cAMP EC₂₀₀/cGMP EC₂₀₀ that is comparable (i.e., varies by no more than 50%) to the value for papaverine.

The assay method of the present invention can use alternative standard cell preparation protocols (Misgeld and Dietzel, Brain Res. 492: 149-57, 1989; Shi and Rayport, J. Neurosci. 14: 4548-60, 1994; Mao and Wang, Mol. Brain. Res. 86: 125-37, 2001; Snyder et al., J. Neuroscience 20: 4480-88, 2000), alternative standard methods of stimulating cyclic nucleotide formation (Svenningsson et at., Neuroscience 84: 223-28, 1998; Glass and Felder, J. Neurosci. 17: 5327-33, 1997), and/or alternative standard methods for cyclic nucleotide detection (Villegas and Brunton, Analytical Biochem. 235: 102-3, 1996; Corbin et al., Methods Enzymol. 159: 74-82, 1988).

Although the cell-based assay of the present invention can be conducted on test agents for which no prior information is known regarding PDE10A inhibition, it is preferred that the cell-based assay be used as a secondary assay to confirm that agents that are identified as PDE10A inhibitors by *in vitro* tests also inhibit PDE10A at the cellular level. Preferably, the agents are identified as PDE10A selective inhibitors. As an alternative to *in vitro* prescreening, agents can also be confirmed as PDE10A inhibitors by *in vitro* testing after identification in the cell based assay.

For *in vitro* tests, agents would be identified as PDE10A selective inhibitors if the IC₅₀ for inhibition of PDEs other than PDE10A were at least 10-fold greater than the IC₅₀ for PDE10A inhibition. To achieve comparable IC₅₀ values, all of the PDE assays are conducted at cyclic nucleotide substrate concentrations which are equivalently proportional to the Km of the cydic nucleotide for each enzyme. One type of screen to identify PDE10A selective modulators uses native enzymes isolated from tissue or recombinant enzymes isolated from transfected host cells, for example, Sf9 insect cells (Fawcett, Proc. Natl. Acad. Sci. USA 97: 3702-07, 2000), yeast cells (Loughney et al., U.S. Pat. No. 5,932,465), or COS-7 cells (Yuasa. J. Biol. Chem. 275: 31469-79, 2000). Preferably, the PDE10A enzyme is human (e.g., Loughney et al.. U.S. Pat: No. 5,932,465, Lanfear et al., EP 967284), mouse (e.g., Lanfear et al., EP 967294), or rat (e.g., SEQ ID NO: 2).

PDE10A activity is measured, for example, as the rate of hydrolysis of an appropriate substrate, [³H]cAMP or [³H]cGMP. This activity is measured, for example, by SPA-based methods (Fawcett, Proc. Natl. Acad. Sci. USA 97: 3702-07, 2000; Phillips et al., WO 00/40733, and Thompson et al., Biochem. 18: 5228, 1979 (as modified using product code TRKQ7090/7100. Amersham Int'l Ltd., Buckhamshire, England)). Briefly, samples containing the PDE10A enzyme are contacted with a cAMP or cGMP substrate (Sigma Chemical), a portion (e.g., ¼ to ½) of which is ³H labelled (Amersham). Reactions are conducted in, for example, microtiter plates (e.g., Microffuor® plates, Dynex Technologies, Chantilly, VA), and are terminated by the addition of yttrium silicate SPA beads (Amersham) containing excess unlabelled cyclic nucleotide. After the beads are allowed to settle in the dark, plates are read by a microtiter plate reader (e.g., TopCount®, Packard. Meriden, CT).

PDE10A activity may also be assayed by detection of ³²P-phosphate released from ³²P-cAMP or ³²P-cGMP (as described, for example, in Loughney et al., J. Biol. Chem. 271: 796-806,1996, and Loughney, U.S. Pat No. 5,932,465), or using antibodies to distinguish between the PDE substrates, cGMP or cAMP, and their hydrotyied products (using, for example AashPlate nil technology, NEN® Life Sciences Products. Boston, MA).

As an alternative to assaying PDE10A catalytic activity, agents can be identified as PDE10A positive modulators or negative modulators (antagonists) if they indirectly modulate PDE10A catalytic activity, for example, via post translational modification (e.g., phosphorylation), modulation of allosteric ligand binding (e.g., via the GAF domain (Fawcett, Proc. Natl. Acad. Sci. USA 97: 3702-07.2000)), or by binding to PDE10A themselves at either a catalytic or allosteric regulatory site. Methods for determining PDE10A phosphorylation and allosteric ligand binding are described in the literature (see, e.g., McAllister-Lucas et al.. J. Biol. Chem. 270: 30671-79, 1995, and Corbin et al., Eur. J. Biochem. 267: 2760-67, 2000).

The test agents used for screening *in vitro* and in the cell-based assay of the present invention may be selected individually or obtained from a compound library. Such agents include peptides, combinatorial chemistry-derived molecular libraries made of D- and/or L-configuration amino acids, phosphopeptides, antibodies, and small organic and inorganic compounds. Libraries include biological libraries, libraries of natural compounds, peptoid libraries (libraries of molecules having the functions of peptides, but with novel, non-peptide backbones which are resistant to enzymatic degradation yet remain bioactive) (see, e.g., Zuckermann. J. Med. Chem. 37: 2678-85, 1994), spatially addressable parallel solid phase or solution phase libraries, synthetic library methods requiring deconvolution, the "one-bead one-compound" library method, and synthetic library methods using affinity chromatography selection.

Examples of methods for the synthesis of molecular libraries can be found in the art, for example, in DeWitt et al., Proc. Nati. Acad. Sci. 90: 6909, 1993; Erd et al.. Proc. Nati. Acad. Sci. 91: 11422, 1994; Zuckermann et al., J. Med. Chem. 37: 2678,1994; Cho et al., Science, 261: 1303, 1995; Carrell et al., Angew. Chem. InL Ed. Engl. 33: 2061, 1994; and Gallop et al.. J. Med. Chem. 37: 1233, 1994.

Libraries of compounds may be presented in solution (e.g., Houghten, Biotechniques, 13: 412-421, 1992), or on beads (Lam, Nature 354: 82-841, 1991), on chips (Fodor, Nature 364: 555-556, 1993), bacteria or spores (Ladner, U.S. Patent No. 5,223,409), plasmids (Cull et at., Proc. Natl. Acad. Sd. USA. 89: 1865-1869, 1992) or on phage (Scott et al., Science 249: 386-390, 1990; Devlin, Science 249: 404-406, 1990; Cwirta et al., Proc. Natt. Acad. Sci. (USA) 87: 6378-6382, 1990; Felid, J. Mol. Biol. 222: 301-310, 1991; Ladner, *supra*).

### The Nucleotide Coding Sequence and Amino Acid Sequence for the Rat PDE10A

The invention encompasses isolated or purified rat PDE10A sequence, for example, as shown in Fig. 1B (SEQ ID NO: 2).

The invention also embraces nucleotide coding sequences that encode a rat PDE10A, for example, as shown in Fig. 1A.

The nucleic acid sequences encoding the rat PDE10A may be extended utilizing a partial nucleotide sequence and employing various PCR-based methods known in the art to detect upstream sequences, such as promoters and regulatory elements. For example, one method which may be employed, restriction-site PCR, uses universal and nested primers to amplify unknown sequence from genomic DNA within a cloning vector (see, e.g., Sarkar, PCR Methods Applic. 2: 318-322, 1993). Another method, inverse PCR, uses primers that extend in divergent directions to amplify unknown sequence from a circularized template. The template is derived from restriction fragments comprising a known genomic locus and surrounding sequences (see, e.g., Triglia et al., Nucleic Acids Res. 16: 8186, 1988). A third method, capture PCR, involves PCR amplification of DNA fragments adjacent to known sequences in human and yeast artificial chromosome DNA (see, e.g., Lagerstrom et al., PCR Methods Applic. 1: 111-119, 1991). In this method, multiple restriction enzyme digestions and ligations may be used to insert an engineered double-stranded sequence into a region of unknown sequence before performing PCR.

In another embodiment of the invention, a polynucleotide of the invention may be cloned in recombinant DNA molecules that direct expression of the rat PDE10A in appropriate host cells. The nucleotide sequences of the present invention can be engineered using methods generally known in the art in order to alter PDE10A-encoding sequences for a variety of purposes including, but not limited to, modification of the cloning, processing, and/or expression of the gene product.

DNA shuffling by random fragmentation and PCR reassembly of gene fragments and synthetic oligonudeotides may be used to engineer the nudeotide sequences. For example, oligonudeotide-mediated site-directed mutagenesis may be used to introduce mutations that create new restriction sites, alter glyoosytation patterns, change codon preference, produce splice variants, and so forth. In another embodiment, sequences encoding a rat PDE10A may be synthesized, in whole or in part, using chemical methods well known in the art (see, e.g., Caruthers et al., Nucleic Acids Symp. Ser. 7: 215-223,1980: and Hom et al., Nucleic Acids Symp. Ser. 7: 225-232, 1980). Alternatively, the rat PDE10A itself or a fragment thereof may be synthesized using chemical methods. For example, peptide synthesis can be performed using various solid-phase techniques (see, e.g., Roberge et al., Science 269: 202-204, 1995). Automated synthesis may be achieved using the ABI 431A peptide synthesizer (Perkin-Elmer, Norwalk, CT). Additionally, the amino acid sequence of rat PDE10A. or any part thereof, may be altered during direct synthesis and/or combined with sequences from other proteins, or any part thereof, to produce a variant polypeptide. The peptide may be substantially purified by preparative high performance liquid chromatography (see, e.g., Chiez and Regnier, Methods Enzymol. 182: 392-421, 1990). The composition of the synthetic peptides may be confirmed by amino acid analysis or by sequencing (see, e.g., Creighton, *Proteins, Structures and Molecular Properties,* WH Freeman, New York, NY 1984).

### Expression Vectors and Host Cells

In order to express a biologically active rat PDE'10A, the nudeotide sequence encoding the rat PDE10A may be inserted into an appropriate expression vector, i.e., a vector which contains the necessary elements for transcriptional and translational control of the inserted coding sequence in a suitable host. These elements include regulatory sequences, such as enhancers, constitutive and inducible promoters, and 5' and 3' untranslated regions derived from the vector and/or from the polynucleotide sequences encoding a rat PDE10A. Such elements may vary in their strength and specificity. Specific initiation signals may also be used to achieve more efficient translation of sequences encoding PDE10A polypeptide. Such signals include the ATG initiation codon and adjacent sequences, e.g. the Kozak sequence. In cases where sequences encoding a rat PDE10A and its initiation codon and upstream regulatory sequences are inserted into the appropriate expression vector, no additional transcriptional or translational control signals may be needed. However, in cases where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used (see, e.g., Scharf et al., Results Probl. Cell Differ. 20: 125-162, 1994). Methods which are well known to those skilled in the art may be used, in light of this disclosure, to construct expression vectors containing sequences encoding a PDE10A polypeptide and appropriate transcriptional and translational control elements. These methods include *in vitro* recombinant DNA techniques, synthetic techniques, and *in vivo* genetic recombination (see, e.g., Sambrook et al., *Molecular Cloning, Laboratory Manual,* Cold Spring Harbor Press. Plainview, NY, chs. 4. 8, and 16-17, 1989; Ausubel et al., *Current Protocols in Molecular Biology,* John Wiley & Sons, New York, NY, chs. 9, 13, and 16, 1995). A variety of expression vector/host systems may be utilized to contain and express sequences encoding a rat PDE10A. These include, but are not limited to, microorganisms such as bacteria transformed with recombinant bacteriophage, plasmid, or cosmid DNA expression vectors; yeast transformed with yeast expression vectors (e.g., episomes, chromosomal elements); insect cell systems infected with viral expression vectors (e.g., baculovirus, paponavirus, *Vaccinia,* adenovirus, pox virus, rabies virus, and retrovirus); plant cell systems transformed with viral expression vectors (e.g., cauliflower mosaic virus, CaMV, or tobacco mosaic virus, TMV), with bacterial expression vectors (e.g., Ti or pBR322 plasmids), or animal cell systems. The invention is not limited by the host cell employed.

In bacterial systems, a number of cloning and expression vectors may be selected depending upon the use intended for polynucleotide sequences encoding the rat PDE10A. For example, routine cloning, subdoning, and propagation of polynucleotide sequences encoding the rat PDE10A polypeptide can be achieved using a multifunctional *E*. *coli* vector such as pBlueScript (Stratagene, La Jolla, CA) or pSport1 plasmid (Life Technologies, Gaithersburg, MD). Ligation of sequences encoding the rat PDE10A polypeptide into the vector's multiple cloning site disrupts the lacZ gene, allowing a colorimetric screening procedure for identification of transformed bacteria containing recombinant molecules. In addition, these vectors may be useful for *in vitro* transcription, dideoxy sequencing, single strand rescue with helper phage, and creation of nested deletions in the cloned sequence (see, e.g., Van Heeke and Schuster, J. Biol. Chem. 264: 5503-5509, 1989). When large quantities of PDE10A polypeptide are needed, e.g., for the production of antibodies, vectors which direct high level expression of PDE10A polypeptide may be used. For example, vectors containing the strong, inducible T5 or T7 bacteriophage promoter may be used.

Recombinant protein expression can be maximized in host bacteria by providing a genetic background wherein the host cell has an impaired capacity to proteotytically deave the recombinant protein (Gottesman et al., Gene Expression Technology: Methods in Enzymology 185: 119-28, 1990). Alternatively, the polynudeotide sequence can be altered to provide preferential codon usage for a specific host cell, e.g., *E*. *coli* (Wada et al., Nucleic Acids Res. 20: 2111-18, 1992).

In yeast expression systems, a number of vectors containing constitutive or inducible promoters, such as alpha factor, alcohol oxidase, or PGH promoters, may be used, for example, in the yeast *Saccharomyces cerevisiae* or *Pichia pastoris.* In addition, such vectors direct either the secretion or intracellular retention of expressed proteins and enable integration of foreign sequences into the host genome for stable propagation (see, e.g., Ausubel, 1995; Bitter et al., Methods Enzymol. 153: 516-544, 1987; and Scorer et al., BioTechnology 12: 181-184, 1994). Plant systems may also be used for expression of the rat PDE10A. Transcription of sequences encoding PDE10A polypeptide may be driven by viral promoters, e.g., the 35S and 19S promoters of CaMV used alone or in combination with the omega leader sequence from TMV (Takamatsu, EMBO J. 6: 307-311, 1987). Alternatively, plant promoters such as the small subunit of RUBISCO or heat shock promoters may be used (see, e.g., Coruzzi et al., EMBO J. 3: 1671-1680, 1984; Broglie et al., Science 224: 838-843, 1984; and Winter et al., Results Probl. Cell Differ. 17: 85-105, 1991). These constructs can be introduced into plant cells by direct DNA transformation or pathogen-mediated transfection (see, e.g., *The McGraw Hill Yearbook of Science and Technology.* McGraw Hill, New York NY, pp. 191-196, 1992).

In mammalian cells, a number of viral-based expression systems may be utilized. In cases where an adenovirus is used as an expression vector, sequences encoding PDE10A polypeptide may be ligated into an adenovirus transcription/translation complex consisting of the late promoter and tripartite leader sequence. Insertion in a non-essential E1 or E3 region of the viral genome may be used to obtain infective virus that expresses rat PDE10A in infected host cells (see, e.g., Logan and Shenk. Proc. Natl. Acad. Sci. USA 81: 3655-3659, 1984). In addition, transcription enhancers, such as the Rous sarcoma virus (RSV) enhancer, may be used to increase expression in mammalian host cells. SV40 or EBV-based vectors may also be used for high-level protein expression.

HACs, BACs, or YACs may also be employed to deliver larger fragments of DNA than can be contained in and expressed from a plasmid. For example, HACs of about 6 kb to 10 Mb are constructed and delivered via conventional delivery methods (liposomes, polycationic amino polymers, or vesicles) for therapeutic purposes (see, e.g., Harrington et al., Nat. Genet. 15: 345-355, 1997). For long term production of recombinant proteins in mammalian systems, stable expression of the rat PDE10A in cell lines is preferred. For example, sequences encoding the rat PDE10A can be transformed into cell lines using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to a selective agent, and its presence allows growth and recovery of cells which successfully express the introduced sequences. Resistant clones of stably transformed cells may be propagated using tissue culture techniques appropriate to the cell type.

The invention also encompasses vectors in which the nucleic acid sequences described herein are cloned into the vector in reverse orientation, but operably linked to a regulatory sequence that permits transcription of antisense RNA. Thus, an antisense transcript can be produced to all, or to a portion, of the nucleic acid molecule sequences described herein, including both coding and noncoding regions. Expression of this antisense RNA is subject to each of the parameters described above in relation to expression of the sense RNA.

Any number of selection systems may be used to recover transformed cell lines. These include, but are not limited to, the herpes simplex virus thymidine kinase and adenine phosphoribosyltransferase genes, for use in TK⁻, and APR⁻- cells, respectively (see, e.g., Wigier et al., Cell 11: 223-232, 1997; Lowy et at, Cell 22:817-823,1980). Also, antimetabolite, antibiotic, or herbicide resistance can be used as the basis for selection. For example, Dhfr confers resistance to methotrexate; Neo confers resistance to the aminoglycosides neomycin and G-418; and Als and Pat confer resistance to chlorsulfuron and phosphinotricin acetyltransferase, respectively (see, e.g., Wigler et al., Proc. Natl. Acad. Sci. USA 77: 3567-3570, 1980; Colbere-Garapin et al., J. Mol. Biol. 150: 1-14, 1981). Additional selectable genes have been described, e.g., TrpB and HisD, which alter cellular requirements for metabolites (see, e.g., Hartman and Mulligan, Proc. Natl. Acad. Sci. USA 85: 8047-8051, 1988). Visible markers, e.g., anthocyanins, green fluorescent proteins (GFP; Clontech, Palo Alto, CA), β-glucuronidase and its substrate β-glucuronide, or luciferase and its substrate luciferin may be used. These markers can be used not only to identify transformants, but also to quantify the amount of transient or stable protein expression attributable to a specific vector system (see, e.g., Rhodes, Methods Mol. Biol. 55: 121-131, 1995). Although the presence/absence of marker gene expression suggests that the gene of interest is also present, the presence and expression of the gene may need to be confirmed. For example, if the sequence encoding the rat PDE10A is inserted within a marker gene sequence, transformed cells containing sequences encoding PDE10A polypeptide can be identified by the absence of marker gene function. Alternatively, a marker gene can be placed in tandem with a sequence encoding PDE10A polypeptide under the control of a single promoter. Expression of the marker gene in response to induction or selection usually indicates expression of the tandem PDE10A sequence as well.

In general, host cells that contain the nucleic acid sequence encoding the rat PDE10A and that express the rat PDE10A may be identified by a variety of procedures known to those of skill in the art. These procedures include, but are not limited to, DNA-DNA or DNA-RNA hybridizations, PCR amplification, and protein bioassay or immunoassay techniques which include membrane, solution, or chip based technologies for the detection and/or quantification of nucleic acid or protein sequences.

Immunological methods for detecting and measuring the expression of PDE10A using either specific polyclonal or monoclonal antibodies are known in the art. Examples of such techniques include enzyme-linked immunosorbent assays (ELISAs), radioimmunoassays (RIAs), Western blots, immunoprecipitation, immunofluorescence, and fluorescence activated cell sorting (FACS). These and other assays are well known in the art (see, e.g., Hampton. *Serological Methods, A Laboratory Manual.* APS Press, St. Paul, MN, Sect. IV, 1990; Coligan et al., *Current Protocols in Immunology,* Greene Pub. Associates and Wiley-Interscience, New York NY, 1997; and Pound, *Immunochemical Protocols,* Humana Press, Totowa, NJ, 1998). A wide variety of labels and conjugation techniques are known by those skilled in the art and may be used in various nucleic acid and amino acid assays.

Means for producing labelled hybridization or PCR probes for detecting sequences related to polynucleotides encoding the rat PDE10A include oligolabelling, nick translation, end-labelling, or PCR amplification using a labelled nucleotide.

Attematively, the sequences encoding the rat PDE10A, or any fragments thereof, may be cloned into a vector for the production of an mRNA probe. Such vectors are known in the art, are commercially available, and may be used to synthesize RNA probes *in vitro* by addition of an appropriate RNA polymerase such as T7, T3, or SP6 and labelled nucleotides. These procedures may be conducted using a variety of commercially available kits (e.g., Amersham Pharmacia Biotech, Piscataway, NJ; Promega, Madison, WI; and US Biochemical, Cleveland, OH). Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding PDE10A polypeptide may be cultured under conditions suitable for the expression and recovery of the protein from cell culture. The protein produced by a transformed cell may be secreted or retained intracellularly depending on the sequence and/or the vector used. As will be understood by those of skill in the art, expression vectors containing polynucleotides which encode the rat PDE10A may be designed to contain signal sequences which direct secretion of the rat PDE10A through a prokaryotic or eukaryotic cell membrane. In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a ''prepro" or "pro" form of the protein may also be used to specify protein targeting, folding, and/or activity.

Different host cells which have specific cellular machinery and characteristic mechanisms for post-translaflonal activities (e.g., CHO, HeLa, MDCK, HEK293, and W138) are available from the American Type Culture Collection (ATCC. Manassas, VA) and may be chosen to ensure the correct modification and processing of the foreign protein. In another embodiment of the invention, natural, modified, or recombinant nucleic acid sequences encoding PDE10A polypeptide may be ligated to a heterologous sequence resulting in translation of a fusion protein in any of the aforementioned host systems. For example, a chimeric PDE10A protein containing a heterologous moiety that can be recognized by a commercially available antibody may facilitate the screening of peptide libraries for modulators of PDE10A polypeptide activity. Heterologous protein and peptide moieties may also facilitate purification of fusion proteins using commercially available affinity matrices. Such moieties include, but are not limited to, glutathione S-transferase (GST), maltose binding protein (MBP), thioredoxin (Trx), calmodulin binding peptide (CBP), 6-His. FLAG, c-myc, and hemagglutinin (HA). GST, MBP, Trx, CBP, and 6-His enable purification of their cognate fusion proteins on immobilized glufiathione, maltose, phenylarsine oxide, calmodulin, and metal-chelate resins, respectively. FLAG, c-myc, and hemagglutinin (HA) enable immunoaffinity purification of fusion proteins using commercially available monoclonal and polyclonal antibodies that specifically recognize these epitope tags. A fusion protein may also be engineered to contain a proteolytic deavage site located between the PDE10A polypeptide encoding sequence and the heterologous protein sequence, so that PDE10A polypeptide may be cleaved away from the heterologous moiety following purification. Methods for fusion protein expression and purification are discussed in Ausubel (1995, *supra,* ch. 10). A variety of commercially available kits may also be used to facilitate expression and purification of fusion proteins.

In a further embodiment of the invention, synthesis of a radiolabelled rat PDE10A may be achieved *in vitro* using the TNT rabbit reticulocyte lysate or wheat germ extract system (Promega). These systems couple transcription and translation of protein-coding sequences operably associated with the T7, T3, or SP6 promoters. Translation takes place in the presence of a radiolabelled amino acid, for example, ³⁵S-methionine.

Fragments of the rat PDE10A may be produced not only by recombinant means, but also by direct peptide synthesis using solid-phase techniques (see, e.g., Creighton, *supra,* pp. 55-60). Protein synthesis may be performed by manual techniques or by automation. Automated synthesis may be achieved, for example, using the ABI® 431 A peptide synthesizer (Perkin-Elmer). Various fragments of the rat PDE10A may be synthesized separately and then combined to produce the full length molecule.

### Nucleic Acid Arrays

The present invention further provides nucleic acid detection kits, such as arrays or microarrays of nucleic add molecules that are based on the sequence information provided in Figure 1A.

As used herein arrays or microarrays refer to an array of distinct polynudeotides or oligonudeotides synthesized on a substrate, such as paper, nylon, or other type of membrane, fitter, chip, glass slide, or any other suitable solid support. In one embodiment, the microarray is prepared and used according to methods described in Chee et al., U.S. Pat. No. 5,837,832, Chee et al., WO 95/11995, Lockhart et al., Nat. Biotech. 14: 1675-80, 1996, Schena et al., Proc. Natl. Acad. Sci. 93: 10614-19, 1996. Other arrays are produced by the methods described in Brown et al., U.S. Pat. No. 5,807,522, and in Baldeschwieler et al., WO 95/25116.

### Example 1: PDE10A Expression Patterns

PDE10A mRNA has been previously reported within the brain in the striatum, nucleus accumbens, and olfactory turbercle (Lanfear and Robas, EP 0967284). The following experiments confirmed these findings and further characterized PDE10A expression in mice and rats.

### Brain sedioning.

Mouse and rat brains were collected after rapid decapitation, frozen in isopentane on dry ice, and cut into 20 µM sections on a Hacker-Bright cryostat (Hacker Instruments, Fairfield, NJ). Sections from all levels of mouse and rat brain were thaw-mounted onto slides previously coated with Vectabond™ reagent (Vector Laboratories, Burlingame, CA), fixed in 4% paraformaldehyde solution, dehydrated in ethanol, and stored with dessicant in airtight containers at 4°C until use.

### Messenger RNA probe generation.

Plasmid DNA containing a 914 base pair fragment isolated from mouse PDE10A cDNA (corresponding to base pairs 380-1294) was inserted into *E*. *coli* (DH5α), which were grown up in suspension culture. The plasmid DNA was extracted with a Qiagen® Maxi-prep kit (Qiagen, Valencia, CA) and stored until use. For the antisense probe, the plasmid was linearized with Xbal restriction endonuclease. For the sense probe, plasmid was linearized with EcoR1 restriction enzyme. The template DNA was then purified using a Qiaquick® PCR kit (Qiagen) and transcribed and labelled with ³³P-UTP using a Maxiscript™ kit (Ambion, Austin, TX). Antisense probe was generated with T7 polymerase, and sense probe with SP6 polymerase. Final labelled riboprobes were prepared using a G50 QuickSpin™ column (Novagen, Madison, WI), and diluted in commercial hybridization buffer (Novagen).

### In situ hybridization.

Slide-mounted brain sections were incubated in proteinase K (1 µg/ml) for 5 min, rinsed in RNase-free water, suspended in 0.1 M fiethylamine (TEA) at pH 8.0. and acetylated by addition of 0.25% acetic anhydride for 5 min. The probes were applied to the slide-mounted sections in a volume of 10-25 µl/section containing ³³P (0.5-1 x 10⁶ cpm per section). As a control, a small number of mouse brain sections were pretreated with RNase A (20 µg/ml) prior to hybridization. The sections were incubated overnight in a humid environment at 50°C. and then rinsed in 2X SSC, treated with RNase A to destroy single-stranded RNA, washed in a standard series of washes, and dehydrated in a graded series of ethanol solutions. The resulting slides were apposed to β-max film (Amersham, Piscataway, NJ) in standard X-ray cassettes, and exposed for 5-10 days.

Following film exposure, slides were dipped in Kodak® NTB-2 emulsion (Eastman Kodak Co., Rochester, NY) diluted 1:1 with water at 43°C under safelight conditions. The slides were air-dried and exposed for two weeks in total darkness during storage at -20°C. The slides were developed in Kodak® D-19 developer and Kodak® fixer at 19°C. The slides were counterstained with 1% Toluidine Blue, dehydrated in alcohol, and coverslipped.

### PDE10A specific antibody.

An antibody directed against the rat PDE10A polypeptide was generated for immunocytochemistry studies. The full-length rat PDE10A sequence (Figure 1B, SEQ ID NO: 2) with a Gterminal His tag was expressed in Sf9 insect cells according to previously reported methods (Fawcett et al., Proc. Natl. Acad. Sci. USA 97: 3702-07. 2000). A three-step purification using Ni-NTA chromatography, buffer exchange, and anion exchange chromatography yielded a final product that was 95% pure, had the appropriate predicted mass of 97 Kd as determined by mass spectrometry, and had cGMP hydrolysis activity. Purified PDE10A was used to immunize mice and Gonal hybridomas were prepared using standard protocols. An antibody designated 24F3.F11 was chosen for use.

The affinity of the monoclonal 24F3.F11 antibody for human and rat PDE10A was tested using cell lysates from an Sf9 cell line that expressed human PDE10A and an Sf9 cell line that expressed rat PDE10A. The monoclonal 24F3.F11 antibody recognizes rat and human recombinant PDE10A. Lysates from the recombinant cells, as well as control Sf9 cells, were subjected to gel electrophoresis. Blots of the gel were then incubated with the 24F3.F11 antibody, and binding of the 24F3.F11 antibody to the blots was determined. No PDE10A immunoreactivity was observed in the lane containing lysates from Sf9 cells not expressing recombinant protein.

The specificity of the 24F3.F11 antibody for PDE10A was compared to PDEs from the other ten PDE gene families. The 24F3.F11 antibody did not cross react with any other PDE.

### Immunocytochemistry and Western blot.

The expression of PDE10A in different regions of rat brain was determined by Western blotting with the 24F3.F11 antibody and by immunocytochemistry. For Western blot analyses, rats were sacrificed by decapitation and brains were quickly removed and chilled on ice. Different brain regions were identified and microdissected using standard techniques. Brain sections were homogenized in 10 volumes of buffer (250 mM NaCl, 50 mM Tris HCl, pH 7.5, 5 mM EDTA, 0.1% NP-40) in a glass/glass homogenizer. Lysates were centrifuged at 4000 rpm (Eppendorf, Hamburg, Germany, model 5417R) and supernatants were subjected to Western blot analyses using standard protocols.

For immunocytochemistry, rat brains were immersion fixed in 10% buffered formalin (pH 7.0) for 24 hours and then imbedded in paraffin. Sections (6 µM) were deparaffinized and hydrated in distilled water. Masked epitopes were retrieved using a citrate buffer (pH 6) heated to 96°C for 20 min. Sections were incubated at room temperature with the 24F3.F11 antibody (1.2 µg/ml) for 60 min. As controls, an IgG1 isotype control antibody and the 24F3.F11 antibody preabsorbed with a saturating concentration (as determined by Western blot) of PDE10A were incubated in parallel on replicate sections. Positive staining was detected using avidin-biotin-HRP complex and visualized with diaminobenzidine (DAB). No reaction product was observed in IgG1 and preabsorbed F11 controls.

### PDE10A mRNA localization.

Autoradiographs of the PDE10A antisense-labelled mouse brain sections displayed a highly specific hybridization signal. Dense labelling was found in only three areas; the dorsal striatum (caudate and putamen), ventral striatum (nucleus accumbens), and olfactory tubercle. Within the striatum and nucleus accumbens, PDE10A mRNA was highly expressed in the striatal medium spiny neurons, which represent about 95% of all neurons found in these structures. A lower density of labelling was noted in dentate gyrus and CA layers of hippocampus and in the granule cell layer of cerebellum. No significant difference was seen in the pattern of labelling in rat brain relative to the mouse.

*In situ* labelling of PDE10A was specific. Mouse brain sections pretreated with RNase A prior to hybridization did not have any visible incorporation of label.

There was very good correspondence between PDE10A mRNA localization areas and those areas classically associated with high dopamine receptor expression. This similarity was further supported by emulsion autoradiographs, which afford subcellular localization and increased resolution relative to film autoradiography. Dense incorporation of silver grains was noted throughout the striatum, nucleus accumbens, and olfactory tubercle, and was noted to overlay the vast majority of the neuronal cell bodies in these three areas. In addition, areas which express low but measurable levels of dopamine receptors also demonstrated grain deposition, in rough correspondence with their relative DA receptor density. These included notably the medial and sulcal prefrontal cortices, as well as dentate gyrus and the CA layers of hippocampus. However, no grain accumulation was seen in the substantia nigra pars reticulata, an area of dense dopamine D1 receptor expression.

### PDE10A protein localization.

Consistent with mRNA levels, a high level of PDE10A protein was demonstrated in the striatum (caudate and putamen), nucleus accumbens, and olfactory tubercle. PDE10A protein was observed in the neuronal cell bodies and throughout the neuropil. Furthermore, a high level of PDE10A protein, but not PDE10A mRNA, was observed in the brain regions to which the striatal medium spiny neurons project, including the internal capsule, globus pallidus, entopeduncular nucleus, and the substantia nigra. Given the absence of PDE10A mRNA in these regions, the high level of PDE10A protein must arise from the axons and terminals of the striatal medium spiny neurons.

### Example 2: In vitro Screening for PDE10A Selective Modulators

Papaverine was identified by *in vitro* testing as a PDE10A selective inhibitor. Papaverine was tested for its ability to inhibit PDE10A as compared to PDEs from the other gene families. Human PDEs 2, 3, and 5 were isolated from corpus cavemosum, human PDE1 was isolated from cardiac ventride, and human PDE4 was isolated from skeletal musde (Bodlell et al., Int. J. Impotence Research 8: 7-52,1896). Phosphodiesterases 7-11 were generated from full length recombinant clones transfected into SF9 cells as previously described (Fisher et al., Biochem. Biophys. Res. Comm. 246: 570-577, 1998; Fisher et al., J. Biol. Chem. 273: 15559-15564, 1998b; Soderling et al., PNAS 96: 7071-7076, 1999; Fawcett et al., PNAS 97: 3702-3707, 2000). The enzymes were purified by FPLC from the soluble fraction of cell lysates as described in Boolell et al., *supra.*

PDE activity was measured using a SPA-based method as previously described (Fawcett et al., 2000). Assays were conducted using a fixed amount of PDE10A enzyme in the presence of varying inhibitor concentrations. The cyclic nucleotide substrates (cGMP or cAMP in a 3:1 ratio of unlabelled to [³H]-labelled) used in the assays were 1/3 of the Km concentration, allowing for comparisons in inhibitor IC₅₀ values for the different PDEs. The final assay volume was adjusted to 100 µl with assay buffer (20 mM Tris-HCl pH 7.4, 5 mM MgCl₂, 1 mg/ml bovine serum albumin).

Reactions were initiated with the addition of enzyme. Samples were incubated for 30-60 min. at 30°C to give <30% substrate tumover and terminated with 50 µl yttrium silicate SPA beads (Amersham) containing 3 mM of the appropriate unlabelled cyclic nucleotide. Plates were re-sealed and shaken for 20 min.; the beads were then allowed to settle for 30 min. in the dark and then counted on a TopCount plate reader (Packard, Meriden, CT). Radioactivity units were converted to percent activity as compared to an uninhibited control (100%). IC₅₀ values were obtained using the 'Fit Curve' Microsoft Excel extension.

Papaverine was identified as a competitive inhibitor of PDE10A, with an IC₅₀ value of 17 nM. Papaverine was considerably less potent against all other PDEs tested (Table 1), demonstrating selectivity for PDE10A.

**Table 1. Papaverine Inhibition of Various PDE Gene Families**

| Isozyme | IC₅₀ (µM) | Selectivity |
|---|---|---|
| PDE10A | 0.018 | 1.0 |
| PDE1 | 37 | 2,055 |
| PDE2 | 9 | 500 |
| PDE3A | 1.3 | 72 |
| PDE4A | 1.9 | 105 |
| PDE4B | 1.4 | 78 |
| PDE4C | 0.8 | 44 |
| PDE4D | 0.32 | 18 |
| PDE5 | 8 | 444 |
| PDE7 | 27 | 1,500 |
| PDE8A | >10 | >555 |
| PDE9 | 400 | 20,000 |
| PDE11 | 11 | 611 |

### Example 3: Primary Striatal Medium Spiny Neurons - Effects of Papaverine

Given the specificity of papaverine for inhibiting PDE10A, papaverine was administered to cultured primary striatal medium spiny neurons to determine if selective PDE10A inhibition had a unique effect on cyclic nucleotide metabolism in these cells. The effects of papaverine were compared to the effects resulting from the administration of other PDE inhibitors, 3-isobutyl-1-methylxanthine (IBMX), rolipram, and zaprinast (all PDE inhibitors available from Sigma, St. Louis, MO).

Striatal cultures were prepared as previously described (Ventimiglia et al., Eur. J. Neurosci. 7: 213-222, 1995). Briefly, striata (caudate nucleus and putamen) were dissected from E17 rats, dissociated to produce a single cell suspension, and plated at a density of 5x10⁴ neurons/well in 96-well plates coated with poly-L-omithine/laminin (Cat. No. 354657, BD Biosciences, Bedford, MA). The cells were plated in Neurobasal medium (Cat. No. 21103-049, Gibco BRL, Grand island, NY) with B27 supplements (Cat. No. 17504-010, Gibco BRL) and human recombinant brain-derived neurotrophic factor (BDNF) (100 ng/ml) (Cat. No. 248-BD, R & D Systems, Minneapolis, MN). Striatal medium spiny neurons comprised 50-60% of cells in these cultures, as confirmed by a GABA immunoreactivity protocol as previously described (Ventimiglia et al., 1995, *supra*). Expression of PDE10A mRNA in these cultures was confirmed by RNase protedion assay, as further described below.

After approximately four days *in vitro,* the striatal cells were washed with Ca²⁺/Mg²⁺ free phosphate buffered saline (pH 7.4) and preincubated for an hour in a buffer containing Ca²⁺/Mg²⁺ free phosphate buffered saline, 30mM HEPES (pH 7.4), 1 mM CaCl₂, 1 mg/ml dextrose, and 5 mM MgCl₂. The striatal cells were then exposed to one of the PDE inhibitors and incubated for 20 min. at 37°C. When measuring cGMP, the neurons were stimulated with sodium nitroprusside (SNP) (100 µM) for two min. after the 20 min. incubation with the PDE inhibitor. When measuring cAMP, the neurons were stimulated with forskolin (1 µM) for the duration of the 20 min. inhibitor incubation. These concentrations of SNP and forskolin were chosen as submaximal concentrations that produced approximately 20-25% of the maximal response (1000 µM SNP and 10 µM forskolin produced maximal increases in cGMP and cAMP, respectively).

cGMP and cAMP SPA systems (Amersham code RPA 540 and RPA 559, respectively) were used to detect the respective cGMP and cAMP concentrations in the cell lysate. The cells were lysed using a 9:1 combination of SPA direct screening Assay Buffer (0.05 M acetate with 0.01% sodium azide) and Buffer A (133 mg/ml dodecyltrimethylammonium bromide), and the lysates were frozen on dry ice.

In cells unstimulated by SNP or forskolin, papaverine did not affect either the cAMP or cGMP levels in the striatal cultures. In the absence of a PDE inhibitor, the submaximal concentrations of forskolin (1 µM) and SNP (100 µM) caused a 2-3 fold increase over basal cAMP and cGMP. Papaverine caused a concentration-dependent increase in SNP-induced cGMP accumulation with an EC₂₀₀ (concentration of the inhibitor yielding a 2-fold increase) value of 11.7 µM (Table 2). A maximal effect was observed at 100 µM papaverine; cGMP levels were elevated 5-fold over cultures stimulated with SNP alone. Papaverine also caused an increase in cAMP accumulation in forskolin-stimulated cultures with an EC₂₀₀ of 38.3 (Table 2). Thus, papaverine was 3.3-fold less potent at promoting an increase in cAMP than cGMP, as determined by the ratio of cGMP EC₂₀₀/cAMP EC₂₀₀ (Table 2).

By contrast, IBMX, a nonselective PDE inhibitor, caused a concentration dependent (over a range of 3-100 µM) increase in both cGMP and cAMP accumulation in SNP- or forskolin-stimulated cultures with EC₂₀₀ values of 19 and 30 µM, respectively. The selective PDE4 inhibitor rolipram increased forskolin stimulated cAMP accumulation with an EC₂₀₀ value of 2.5 µM and required 10-fold higher concentrations to double the rate of cGMP accumulation. Zaprinast, an inhibitor of cGMP-preferring PDEs, doubled the cAMP levels in these neurons at a concentration of 98 µM. However, 100 µM of this compound did not quite double the level of cGMP. Comparisons of the ratios of cGMP EC₂₀₀/cAMP EC₂₀₀ for each PDE inhibitor are shown in Table 2 and demonstrate that PDE10A selective inhibitors (as represented by papaverine) have a unique effect on cyclic nucleotide regulation in striatal medium spiny neurons.

**Table 2. Comparison of PDE Inhibitors**

| PDE inhibitor | cGMP EC₂₀₀ µM±SEM (n) | cAMP EC₂₀₀ µM±SEM (n) | cAMP EC₂₀₀/cGMP EC₂₀₀ |
|---|---|---|---|
| Papaverine | 11.7±8.2(4) | 38.3±11.4 (4) | 3.3 |
| Rolipram | 29.2±10.3 (3) | 2.5±2.0 (3) | 0.09 |
| Zaprinast | 98.3±10.3 (3) | >100 (3) | 1 |
| IBMX | 19.5 (1) | 30.2(2) | 1.5 |

### RNase protection assay.

RNA was prepared from a primary culture of rat striatal medium spiny neurons by centrifugation at 150,000 x g at 20°C for 21 hours through a 5.7 M cesium chloride gradient as previously described (Iredale PA, et al., Mol. Pharmacol. 50: 1103-1110, 1996). The RNA pellet was resuspended in 0.3 M sodium acetate, pH 5.2, and precipitated in ethanol. The RNA concentration was determined by spectrophotometry. A PDE10A riboprobe was prepared by PCR amplification of a 914 bp fragment isolated from mouse cDNA (corresponding to base pairs 380-1294 of Genbank AF110507). This fragment was then cloned into pGEM3Zf. The vector was linearized and T7 RNA polymerase was used to synthesize [³²P]-labelled antisense riboprobe.

The RNase protection assay was performed using the RPAII kit (Ambion). Briefly, 5 µg of total cellular RNA was hybridized with [³²P]-labelled PDE10A riboprobe (approximately 105 cpm/sample) overnight at 42°C. The following day the samples were incubated with RNase A and T1 for 30 min. at 37°C and the protected double-stranded RNA fragments were then precipitated and run on a 6% polyacrylamide gel containing urea. The results of this assay confirmed that PDE10A mRNA was present in a high level in the primary culture of striatal medium spiny neurons.

### Example 4: Cloning and Sequencing of Rat PDE10A

The protein coding sequence of human PDE10A (GenBank AB020593) was used to search a selected subset of the National Center for Biotechnology Information (NCBI) Expressed Sequence Tag (EST) Database containing non-human ESTs using Basic Local Alignment Search Tool (BLAST) (Altshul et al., J. Mol. Bio. 215, 403-410, 1990). The amino acid sequence predicted by one rat EST (H32734) was homologous to an internal portion of the human protein. In addition, the nucleic acid sequence of this EST was highly homologous to that of mouse PDE10A (GenBank AF110507). We used this EST information plus 5' and 3' Rapid Amplification of cDNA Ends (RACE) techniques to identify authentic 5' and 3' sequence from rat brain RNA, which then was used to isolate a complete rat PDE10A cDNA.

5' RACE was performed using a 5' RACE kit (Gibco/BRL) according to manufacturer's recommendations. First strand cDNA was generated using gene specific 3' primer 1358 (Table 3), 1 µg total RNA from Sprague-Dawley rat brain, and Superscript™ II Reverse Transcriptase (Gibco/BRL). First round PCR was carried out using nested 3' gene specific primer 1357 (Table 3) and 5' adapter primer (Abridged Anchor Primer (AAP)) from the kit Cycling conditions induded an initial 2 min. denaturation at 94°C followed by 35 cydes of 94°C denaturation for 45 sec., 54°C anneal for 30 sec., 72°C extension for 3 min., plus a final 10 min. extension at 72 °C (Robocycler®, Stratagene, La Jolla, CA).

First round PCR product (1 µl) was used as template in a second round of PCR using the above cycling conditions, with nested gene specific primer 1356 (Table 3) and the 5' adapter primer (Abridged Universal Amplification Primer (AUAP)) from the kit Six separate PCR products were identified by electrophoresis on 1.2% agarose gels. These bands were individually isolated using a gel extraction kit (Qiagen, Valencia, CA), doned into pCR4 TOPO (Invitrogen. Carlsbad, CA) and transfected into TOP10 cells (Invitrogen). Colonies were screened by PCR with vector specific primers 1369 and 1370 (Table 3) as described above and multiple positive clones from each band were sequenced.

All sequences were aligned to produce a consensus sequence for the 5' end of rat PDE10A. 3' RACE was performed using a 3' RACE kit (Gibco/BRL) according to manufacturer's instructions. First strand cDNA was generated from 2 µg rat brain total RNA using the 3' adapter primer (AP) and Superscript® II Reverse Transcriptase. First round PCR was carried out using gene specific 5' primer 1375 (Table 3) and the 3' AP from the kit. Cycling conditions were as described above. First round PCR product (1 µl) was used as template in a second round of PCR, using the same cycling conditions, with the nested primer 1376 (Table 3) and 3' AUAP. Four discrete PCR products of the approximate expected size were identified by eledrophoresis on 1.2% agarose gels. These bands were isolated and subcloned and colonies screened as above. Positive clones were identified from one PCR product. The sequences from six of these clones were aligned to produce a consensus sequence for the 3' end of rat PDE10A.

To isolate full length rat PDE10A cDNA, first strand cDNA was generated in triplicate from total rat brain RNA using the Superscript® System (Gibco/BRL). 5' Primer 1380 and 3' primer 1407 (Table 3) were designed from rat 5' and 3' sequence information generated above and engineered to include flanking Sall restriction sites to facilitate doning. PCR was performed using a High Fidelity PCR System (Boehringer Mannheim (Roche), Basel, Switzerland). Cycling conditions were an initial 2 min. of denaturation at 94°C, followed by 4 cycles of 94°C denaturation for 45 sec., 48°C anneal for 30 sec., 72°C extension for 3.5 min., then 30 cycles of 94°C denaturation for 45 sec., 55°C anneal for 30 sec., 72°C extension for 3.5 min., plus a final 10 min. extension at 72°C (Robocycler®, Stratagene). Separate PCR reactions were carried out for each of the three first strand cDNA templates.

A band of approximately 2.3 kb from each PCR reaction was gel isolated using a gel extraction kit (Qiagen, Valencia, CA) and ligated into PCR Blunt (Invitrogen) and transformed as above. Correct clones were identified by colony PCR using rat PDE10 specific primers 1385 and 1386 (Table 3) and Sall restriction digest Multiple dones from each separate reverse transcription PCR reaction were sequenced, and all sequences aligned to determine a consensus. Two error free clones (pNB1426 and pNB1427) were identified and sequenced. The polynucleotide sequence (SEQ ID NO: 1) and predicted amino acid sequence (SEQ ID NO: 2) for rat PDE10A are shown in Fig. 1A and Fig. 1B, respectively.

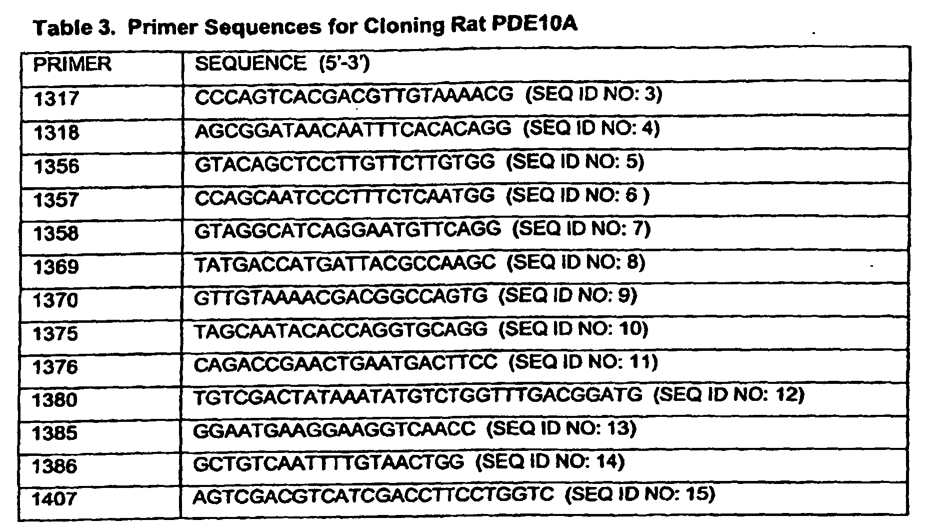

## Claims

1. A method of screening for an agent that inhibits intracellular phosphodiesterase 10A activity, said method comprising:
i) administering the agent to striatal medium spiny neurons and submaximally activating adenylate cyclase;
ii) administering the agent to striatal medium spiny neurons and submaximally activating guanylate cyclase;
iii) measuring cAMP generation in the cells of step (i) and cGMP generation in the cells of step (ii);
iv) calculating the cAMP EC₂₀₀ for step (i) and the cGMP EC₂₀₀ for step (ii);
wherein the agent is identified as a PDE10A inhibitor if the ratio of cAMP EC₂₀₀/ cGMP EC₂₀₀ is comparable to the ratio produced by administration of papaverine under the same assay conditions.

2. The method of claim 1, wherein said striatal medium spiny neurons are prepared as primary cultured neurons.

3. The method of claim 2, wherein adenylate cyclase is activated by forskolin, guanylate cyclase is activated by sodium nitroprusside, and the cAMP EC₂₀₀/cGMP EC₂₀₀ ratio ranges from 1.75-5.25.

4. The method of claim 3, wherein said cAMP EC₂₀₀/cGMP EC₂₀₀ ratio ranges from 3.0-4.0.

5. The method of claim 1, wherein the concentration of CAMP and cGMP is measured by scintillation proximity assay.

6. The method of claim 1, wherein, prior to steps (i) and (ii), said agent is identified *in vitro* as a PDE10A selective inhibitor.

7. The method of claim 1, wherein said agent is further identified as a PDE10A selective inhibitor by *in vitro* assay.

8. The method of claim 1, wherein the neurons of steps (i) and (ii) are in separate samples.

9. An isolated or purified polypeptide comprising the amino acid sequence of SEQ ID NO: 2.

10. An isolated or purified polynucleotide comprising:
i) a nucleic acid sequence encoding the polypeptide of SEQ ID NO: 2; or
ii) the coding sequence of SEQ ID NO: 1.

11. A vector comprising a polynucleotide of claim 10.

12. A host cell transformed with a vector of claim 11 and expressing a polynucleotide of claim 10.

13. A method of identifying an agent that modulates PDE10A activity, said method comprising contacting said agent with a PDE10A polypeptide comprising SEQ ID NO:2 and measuring the activity of said PDE10A polypeptide, wherein a difference between said PDE10A polypeptide activity in the presence of the agent and in the absence of the agent is indicative that the agent modulates said activity.

14. A method of identifying an agent that modulates PDE10A activity, said method comprising contacting said agent with a host cell of claim 12 and measuring the activity of said PDE10A polypeptide, wherein a difference between said PDE10A polypeptide activity in the presence of the agent and in the absence of the agent is indicative that the agent modulates said activity.

## Revendications

1. Procédé de criblage tendant à identifier un agent qui inhibe l'activité de la phosphodiestérase intracellulaire 10A, ledit procédé comprenant .
i) l'administration de l'agent à des neurones épineux de taille moyenne du striatum et l'activation de l'adénylate cyclase à un niveau sous-maximal ;
ii) l'administration de l'agent à des neurones épineux de taille moyenne du striatum et l'activation de la guanylate cyclase à un niveau sous-maximal ;
iii) la mesure de la production d'AMPc dans les cellules de l'étape (i) et de la production de GMPc dans les cellules de l'étape (ii) ;
iv) le calcul de la CE₂₀₀ pour l'AMPc pour 1 ' étape (i) et de la CE₂₀₀ pour la GMPc pour l'étape (ii) ;
procédé dans lequel l'agent est identifié comme étant un inhibiteur de la PDE10A si le rapport CE₂₀₀ AMPc/CE₂₀₀ GMPc est comparable au rapport produit par l'administration de papavérine dans les mêmes conditions de test.

2. Procédé selon la revendication 1, dans lequel les neurones épineux de taille moyenne du striatum sont préparés sous la forme de neurones de culture primaire.

3. Procédé selon la revendication 2, dans lequel l'adénylate cyclase est activée par la forskoline, la guanylate cyclase est activée par le nitroprussiate de sodium et le rapport CE₂₀₀ AMPc/CE₂₀₀ GMPc est compris entre 1,75 et 5,25.

4. Procédé selon la revendication 3, dans lequel ledit rapport CE₂₀₀ AMPc/CE₂₀₀ GMPc est compris entre 3, 0 et 4,0.

5. Procédé selon la revendication 1, dans lequel la concentration en AMPc et GMPc est mesurée par comptage par scintillation de proximité.

6. Procédé selon la revendication 1, dans lequel, avant les étapes (i) et (ii), ledit agent est identifié *in vitro* comme étant un inhibiteur sélectif de la PDE10A.

7. Procédé selon la revendication 1, dans lequel ledit agent est en outre identifié comme étant un inhibiteur sélectif de la PDE10A par un essai *in vitro.*

8. Procédé selon la revendication 1, dans lequel les neurones des étapes (i) et (ii) sont dans des échantillons séparés.

9. Polypeptide isolé ou purifié comprenant la séquence d'acides aminés SEQ ID NO: 2.

10. Polynucléotide isolé ou purifié comprenant .
(i) une séquence d'acide nucléique codant pour le polypeptide de séquence SEQ ID NO: 2 ; ou
(ii) la séquence codante SEQ ID NO: 1.

11. Vecteur comprenant un polynucléotide selon la revendication 10.

12. Cellule hôte transformée au moyen d'un vecteur selon la revendication 11 et exprimant un polynucléotide selon la revendication 10.

13. Procédé d'identification d'un agent qui module l'activité de la PDE10A, ledit procédé comprenant la mise en contact dudit agent avec un polypeptide PDEIOA comprenant la séquence SEQ ID NO: 2 et la mesure de l'activité dudit polypeptide PDE10A, procédé dans lequel une différence entre ladite activité du polypeptide PDE10A en présence de l'agent et en l'absence de l'agent indique que l'agent module ladite activité.

14. Procédé d'identification d'un agent qui module l'activité de la PDE10A, ledit procédé comprenant la mise en contact dudit agent avec une cellule hôte selon la revendication 12 et la mesure de l'activité dudit polypeptide PDE10A, procédé dans lequel une différence entre ladite activité du polypeptide PDE10A en présence de l'agent et en l'absence de l'agent indique que l'agent module ladite activité.

## Patentansprüche

1. Verfahren zum Screening nach einem Agens, das intrazelluläre Phosphodiesterase-10A-Aktivität hemmt, wobei das Verfahren umfasst:
i) Verabreichung des Agenses an mittelgroße Projektionsneuronen des Striatum und submaximale Aktivierung von Adenylatcyclase;
ii) Verabreichung des Agenses an mittelgroße Projektionsneuronen des Striatum und submaximale Aktivierung von Guanylatcyclase;
iii) Messung der cAMP-Bildung in den Zellen von Schritt (i) und der cGMP-Bildung in den Zellen von Schritt (ii);
iv) Berechnung des cAMP-EC₂₀₀-Wertes für Schritt (i) und des cGMP-EC₂₀₀-Wertes für Schritt (ii);
wobei das Mittel als ein PDE10A-Inhibitor identifiziert wird, wenn das Verhältnis cAMP-EC₂₀₀-Wert/cGMP-EC₂₀₀-Wert mit dem Verhältnis vergleichbar ist, das durch Verabreichung von Papaverin unter denselben Assay-Bedingungen produziert wird.

2. Verfahren nach Anspruch 1, wobei die mittelgroßen Projektionsneuronen des Striatum als primär kultivierte Neuronen hergestellt werden.

3. Verfahren nach Anspruch 2, wobei Adenylatcyclase durch Forskolin aktiviert wird, Guanylatcyclase durch Natriumnitroprussid aktiviert wird und das Verhältnis cAMP-EC₂₀₀-Wert/cGMP-EC₂₀₀-Wert im Bereich von 1,75-5,25 liegt.

4. Verfahren nach Anspruch 3, wobei das Verhältnis cAMP-EC₂₀₀-Wert/cGMP-EC₂₀₀-Wert im Bereich von 3,0-4,0 liegt.

5. Verfahren nach Anspruch 1, wobei die Konzentration an cAMP und cGMP durch einen Szintillationsnäherungs-Assay gemessen wird.

6. Verfahren nach Anspruch 1, wobei das Agens vor den Schritten (i) und (ii) in vitro als selektiver PDE10A-Inhibitor identifiziert wird.

7. Verfahren nach Anspruch 1, wobei das Agens außerdem in einem in-vitro-Assay als selektiver PDE10A-Inhibitor identifiziert wird.

8. Verfahren nach Anspruch 1, wobei die Neuronen von Schritt (i) und (ii) in getrennten Proben vorliegen.

9. Isoliertes oder gereinigtes Polypeptid, das die Aminosäuresequenz von SEQ ID NO:2 umfasst.

10. Isoliertes oder gereinigtes Polypeptid, umfassend:
i) eine Nucleinsäuresequenz, die für das Polypeptid von SEQ ID NO:2 codiert;
ii) die codierende Sequenz von SEQ ID NO:1.

11. Vektor, der ein Polynucleotid nach Anspruch 10 umfasst.

12. Eine Wirtszelle, die mit einem Vektor nach Anspruch 11 transformiert ist und die ein Polynucleotid nach Anspruch 10 exprimiert.

13. Verfahren zur Identifizierung eines Agenses, das PDE10A-Aktivität moduliert, wobei das Verfahren Inkontaktbringen des Agenses mit einem PDE10A-Polypeptid, das SEQ ID NO:2 umfasst, und Messen der Aktivität des PDE10A-Polypeptids umfasst, wobei eine Differenz zwischen der PDE10A-Polypeptid-Aktivität in Gegenwart des Agenses und in Abwesenheit des Agenses ein Hinweis dafür ist, dass das Agens die Aktivität moduliert.

14. Verfahren zur Identifizierung eines Agenses, das PDE10A-Aktivität moduliert, wobei das Verfahren Inkontaktbringen des Agenses mit einer Wirtszelle nach Anspruch 12 und Messen der Aktivität des PDE10A-Polypeptids umfasst, wobei eine Differenz zwischen der PDE10A-Polypeptid-Aktivität in Gegenwart des Agenses und in Abwesenheit des Agenses ein Hinweis dafür ist, dass das Agens die Aktivität moduliert.
